(19) Europäisches Patentamt
European Patent Office
Office européen des brevets

(11) EP 4 421 108 A2

# (12) EUROPEAN PATENT APPLICATION

(43) Date of publication:
**28.08.2024 Bulletin 2024/35**

(21) Application number: **24187386.8**

(22) Date of filing: **11.08.2021**

(51) International Patent Classification (IPC):
***C08G 64/06*** *(2006.01)*

(52) Cooperative Patent Classification (CPC):
(C-Sets available)
**C08G 64/1608; C07D 307/83; C08G 64/305; C08K 5/13; C08K 5/1535; C08K 5/42; C08K 5/526;**
C08K 5/005; C08K 2201/019 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.08.2020 JP 2020136732**
**06.11.2020 JP 2020186035**
**19.03.2021 JP 2021046347**

(62) Document number(s) of the earlier application(s) in accordance with Art. 76 EPC:
**21855977.1 / 4 198 092**

(71) Applicant: **MITSUBISHI GAS CHEMICAL COMPANY, INC.**
**Chiyoda-ku**
**Tokyo 100-8324 (JP)**

(72) Inventors:
- **Kato, Noriyuki**
  **Tokyo, 100-8324 (JP)**
- **Nishimori, Katsushi**
  **Tokyo, 125-8601 (JP)**
- **Ikeda, Shinya**
  **Niigata-shi, Niigata, 950-3121 (JP)**
- **Motegi, Atsushi**
  **Tokyo, 125-8601 (JP)**
- **Murata Suzuki, Shoko**
  **Tokyo, 125-8601 (JP)**
- **Ochi, Noriaki**
  **Tokyo, 125-8601 (JP)**
- **Kanda, Masahiro**
  **Tokyo, 125-8601 (JP)**
- **Nagai, Masayuki**
  **Hiratsuka-shi, Kanagawa, 254-0016 (JP)**
- **Morishita, Takami**
  **Hiratsuka-shi, Kanagawa, 254-0016 (JP)**
- **Ikeda, Shinichi**
  **Hiratsuka-shi, Kanagawa, 254-0016 (JP)**

(74) Representative: **Hoffmann Eitle**
**Patent- und Rechtsanwälte PartmbB**
**Arabellastraße 30**
**81925 München (DE)**

Remarks:
This application was filed on 09-07-2024 as a divisional application to the application mentioned under INID code 62.

## (54) THERMOPLASTIC RESIN COMPOSITION FOR OPTICAL MATERIAL, MOLDED ARTICLE, COMPOUNDING AGENT, METHOD FOR PRODUCING THERMOPLASTIC RESIN COMPOSITION, AND METHOD FOR IMPROVING TRANSMISSIVITY

(57) The present invention relates to a thermoplastic resin composition comprising a compounding agent represented by General formula (1), wherein in General formula (1), $R_1$-$R_5$ each independently represent a hydrogen atom or an optionally substituted alkyl group with a total of 1-20 carbon atoms, $R_6$-$R_9$ each independently represent a hydrogen atom or an optionally substituted alkyl group with a total of 1-20 carbon atoms, and $R_{10}$ represents a hydrogen atom or an alkyl group with a total of 1-5 carbon atoms. The present invention also relates to use of a compounding agent and a method for producing a thermoplastic resin composition.

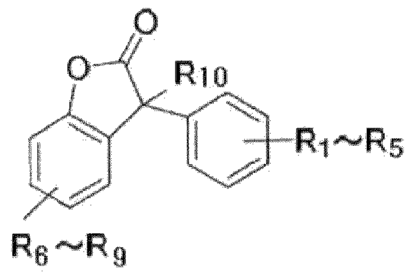

(1)

EP 4 421 108 A2

(52) Cooperative Patent Classification (CPC): (Cont.)

C-Sets
**C08K 5/13, C08L 69/00;**
**C08K 5/1535, C08L 69/00;**
**C08K 5/42, C08L 69/00;**
**C08K 5/526, C08L 69/00**

## Description

TECHNICAL FIELD

The present invention relates to a thermoplastic resin composition and the like. More particularly, the present invention relates to a thermoplastic resin composition for use as an optical material, a molded article containing the thermoplastic resin composition, a compounding agent for the addition to a thermoplastic resin, a method for producing the thermoplastic resin composition, a method for improving transmissivity, and others.

BACKGROUND ART

Conventionally, additives such as an antioxidant and mold release agent are added to a thermoplastic resin which is used as an optical material to ensure stability and mold release property upon processing. For example, addition of an antioxidant to a resin is known to improve stability upon processing (e.g., Patent literature 1 and 2).

However, the addition of such additives sometimes impairs the intrinsic performance of the resin. For example, in a thermoplastic resin which is used an optical material, the addition of an additive has a problem of reducing the transmissivity in the short wavelength range, which is extremely critical.

A slight change in transmissivity in the short wavelength range in a thermoplastic resin as an optical material may have a significant impact on the end product. Therefore, a resin composition that can maintain the original transmissivity of the thermoplastic resin that is used as an optical material after it is made into a commercialized product has been sought, but no such composition that can reliably suppress changes in the transmissivity has been realized.

PRIOR ART DOCUMENTS

PATENT DOCUMENTS

Patent literature 1: Japanese Unexamined Patent Application Publication No.
Patent literature 2: WO99/67232

SUMMARY OF THE INVENTION

PROBLEMS TO BE SOLVED BY THE INVENTION

The main objective of the present invention includes providing a thermoplastic resin composition for use as an optical material, in which changes in transmissivity, especially in the short wavelength range, caused by addition of additives for manufacturing or else, can be suppressed.

MEANS FOR SOLVING THE PROBLEMS

The present inventors have found that a thermoplastic resin composition that has been blended with a certain lactone-based compound maintains good transmissivity, especially in the short wavelength range, even in the presence of additives.

The present invention includes the following.

<1> A thermoplastic resin composition comprising a compounding agent represented by General formula (1) below:

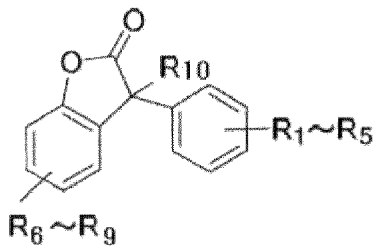

(1)

(in General formula (1),

$R_1$-$R_5$ each independently represent a hydrogen atom or an optionally substituted alkyl group with a total of 1-20 carbon atoms,
$R_6$-$R_9$ each independently represent a hydrogen atom or an optionally substituted alkyl group with a total of 1-20 carbon atoms, and
$R_{10}$ represents a hydrogen atom or an alkyl group with a total of 1-5 carbon atoms.)

<2> The thermoplastic resin composition according to <1> above, further comprising an antioxidant.
<3> The thermoplastic resin composition according to <2> above, wherein the antioxidant is a phenolic antioxidant and/or a phosphite-based antioxidant.
<4> The thermoplastic resin composition according to either one of <2> and <3> above, wherein the antioxidant is contained in an amount of 1-10,000 ppm by weight based on the total weight of the resin composition.
<5> The thermoplastic resin composition according to <4> above, wherein the antioxidant is contained in an amount of 1-3,000 ppm by weight based on the total weight of the resin composition.
<6> The thermoplastic resin composition according to any one of <1> to <5> above, wherein the compounding agent is contained in an amount of 1-10,000 ppm by weight based on the total weight of the resin composition.
<7> The thermoplastic resin composition according to any one of <1> to <6> above, wherein the compounding agent is contained in an amount of 1-2,000 ppm by weight based on the total weight of the resin composition.
<8> The thermoplastic resin composition according to any one of <1> to <7> above, wherein the value of transmissivity (%) at a wavelength of 370-400 nm according to JIS K7105 is higher than that of a reference resin composition having the same composition but without the compounding agent, by 2.0 (%) or more.
<9> The thermoplastic resin composition according to any one of <1> to <8> above, wherein the value of transmissivity (%) at a wavelength of 370-400 nm according to JIS K7105 is 1.1 times or more higher than that of a reference resin composition having the same composition but without the compounding agent.
<10> The thermoplastic resin composition according to any one of <1> to <9> above, wherein the amount of a volatile component generated upon heating at 250°C for 5 minutes is less than that of a reference resin composition having the same composition but without the compounding agent, and
the volatile component is any of formaldehyde, acetaldehyde, acetone, 2,3-butanedione, acetic acid, and formic acid.
<11> The thermoplastic resin composition according to any one of <1> to <10> above, wherein the YI value according to JIS K7105 is lower than that of a reference resin composition having the same composition but without the compounding agent, by 0.20 or more.
<12> The thermoplastic resin composition according to any one of <1> to <11> above, wherein, in General formula (1) above,

three of $R_1$-$R_5$ are hydrogen atoms while two are alkyl groups,
two of $R_6$-$R_9$ are hydrogen atoms while two are alkyl groups, and
$R_{10}$ is a hydrogen atom.

<13> The thermoplastic resin composition according to any one of <1> to <12> above, wherein, in General formula (1) above, the substituent is any of a halogen, a cyano group, an alkenyl group, an alkynyl group, and an alkoxy group.
<14> The thermoplastic resin composition according to any one of <1> to <13> above, further comprising a thermoplastic resin, wherein the thermoplastic resin is selected from the group consisting of a polycarbonate resin, a polyester resin, a polyester carbonate resin, a cyclo-olefin-based resin, and an acrylic resin.
<15> The thermoplastic resin composition according to <14> above, wherein the thermoplastic resin is a polycarbonate resin, a polyester resin, or a polyester carbonate resin, which comprises a structural unit (B) derived from a monomer represented by General formula (2) below and/or a structural unit (C) derived from a monomer represented by General formula (3) below:

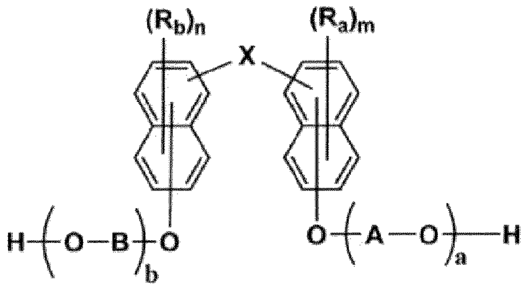

(2)

(in General formula (2),

$R_a$ and $R_b$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 6-20 carbon atoms containing one or more heteroatoms selected from O, N and S, an optionally substituted aryloxy group with 6-20 carbons, and $-C{\equiv}C-R_h$,
$R_h$ represents an optionally substituted aryl group with 6-20 carbon atoms or an optionally substituted heteroaryl group with 6-20 carbon atoms containing one or more heteroatoms selected from O, N and S,
X represents a single bond or an optionally substituted fluorene group,
A and B each independently represent an optionally substituted alkylene group with 1-5 carbon atoms,
m and n each independently represent an integer from 0 to 6, and
a and b each independently represent an integer from 0-10): and

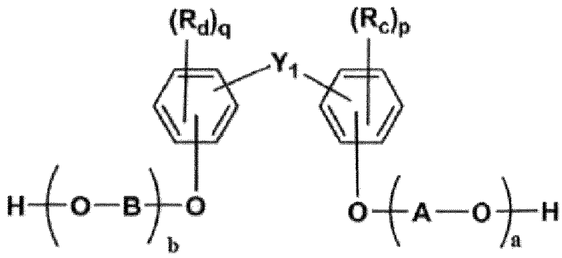

(3)

(in General formula (3),
$R_c$ and $R_d$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, and an optionally substituted aryl group with 6-20 carbon atoms,
A and B each independently represent an optionally substituted alkylene group with 1-5 carbon atoms,
p and q each independently represent an integer from 0 to 4,
a and b each independently represent an integer from 0-10,
$Y_1$ is a single bond, an optionally substituted fluorene group, or any one of the structural formulae represented by the General formulae (4) to (9) and (12) to (14) below:

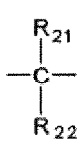

(4)

$-S-$

(5)

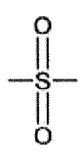

(6)

$-(CH_2)_r-$

(7)

$-O-$

(8)

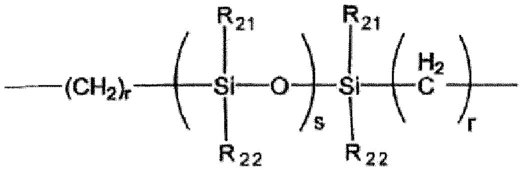 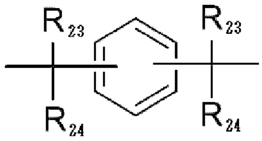 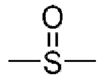 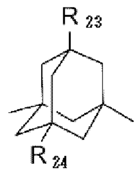

(9) (12) (13) (14)

(in General formulae (4) to (9),

$R_{21}$ and $R_{22}$ each independently represent a hydrogen atom, a halogen atom, an optionally substituted alkyl group with 1-20 carbon atoms, or an optionally substituted aryl group with 6-30 carbon atoms, or an optionally substituted carbon or heterocyclic ring with 1-20 carbon atoms which is formed of $R_{21}$ and $R_{22}$ bonding to each other, and

r and s each independently represent an integer from 0 to 5,000; and

in General formulae (12) to (14),

$R_{23}$ and $R_{24}$ each independently represent a hydrogen atom, fluorine, chlorine, bromine, iodine, an optionally substituted alkyl group with 1-9 carbon atoms, an optionally substituted alkoxy group with 1-5 carbon atoms, an optionally substituted alkenyl group with 2-12 carbon atoms, or an optionally substituted aryl group with 6-12 carbon atoms.)

<16> The thermoplastic resin composition according to either one of <14> and <15> above, wherein the weight-average molecular weight (Mw) of the thermoplastic resin in terms of polystyrene is 10,000-300,000.

<17> The thermoplastic resin composition according to either one of <15> and <16> above, wherein, in General formulae (2) and (3), A and B each independently represent an alkylene group with 2 or 3 carbon atoms.

<18> The thermoplastic resin composition according to any one of <14> to <17> above, wherein the thermoplastic resin at least contains a structural unit derived from any one of BPEF, BNE, BNEF, and DPBHBNA.

<19> The thermoplastic resin composition according to any one of <1> to <18> above, further comprising a catalyst deactivator.

<20> The thermoplastic resin composition according to <19> above, wherein the catalyst deactivator comprises dodecylbenzene sulfonate.

<21> The thermoplastic resin composition according to any one of <1> to <20> above, further comprising a mold release agent.

<22> The thermoplastic resin composition according to <21> above, wherein the mold release agent is contained in an amount of 1-5,000 ppm by weight based on the total weight of the resin composition.

<23> The thermoplastic resin composition according to any one of <1> to <22> above, comprising a compounding agent represented by General formula (1) above, which has peaks at diffraction angles 2θ of 6.7 ± 0.2°, 10.4 ± 0.2°, 11.1 ± 0.2°, 12.7 ± 0.2°, 13.2 ± 0.2°, 15.2 ± 0.2°, 16.1 ± 0.2°, 17.3 ± 0.2°, 20.8 ± 0.2°, and 23.6 ± 0.2° in a powder X-ray diffraction pattern using Cu-Kα radiation.

<24> The thermoplastic resin composition according to any one of <1> to <23> above, which is used as an optical material.

<25> A thermoplastic resin composition comprising a compounding agent represented by General formula (1) below to improve the value of transmissivity (%) at a wavelength of 370 nm-400 nm:

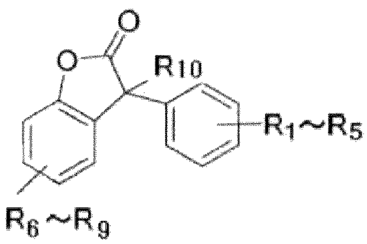

(1)

(in General formula (1),

$R_1$-$R_5$ each independently represent a hydrogen atom or an optionally substituted alkyl group with a total of

1-20 carbon atoms,

$R_6$-$R_9$ each independently represent a hydrogen atom or an optionally substituted alkyl group with a total of 1-20 carbon atoms, and

$R_{10}$ represents a hydrogen atom or an alkyl group with a total of 1-5 carbon atoms.)

<26> A molded article comprising the thermoplastic resin composition according to any one of <1> to <25> above.

<27> A compounding agent represented by Formula (10) or (11) below, which is added to a thermoplastic resin to improve the value of transmissivity (%) of a thermoplastic resin composition at a wavelength of 370 nm-400 nm.

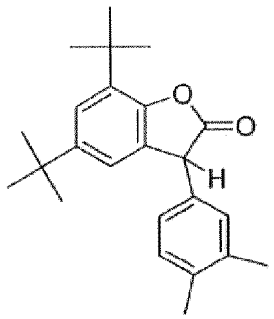
(10)

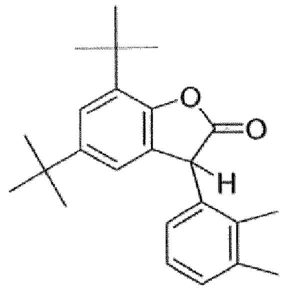
(11)

<28> A compounding agent represented by Formula (10) or (11) below, which is added to a thermoplastic resin to lower the haze value of a thermoplastic resin composition.

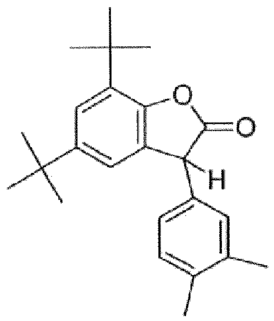
(10)

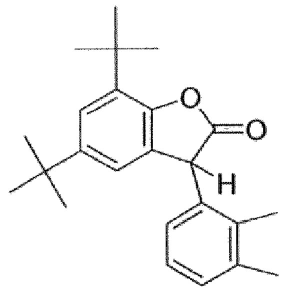
(11)

<29> The compounding agent according to either one of <27> and <28> above, which has peaks at diffraction angles $2\theta$ of 6.7 $\pm$ 0.2°, 10.4 $\pm$ 0.2°, 11.1 $\pm$ 0.2°, 12.7 $\pm$ 0.2°, 13.2 $\pm$ 0.2°, 15.2 $\pm$ 0.2°, 16.1 $\pm$ 0.2°, 17.3 $\pm$ 0.2°, 20.8 $\pm$ 0.2°, and 23.6 $\pm$ 0.2° in a powder X-ray diffraction pattern using Cu-K$\alpha$ radiation.

<30> A method for producing a thermoplastic resin composition, the method comprising a step of adding a compounding agent represented by General formula (1) below to a thermoplastic resin:

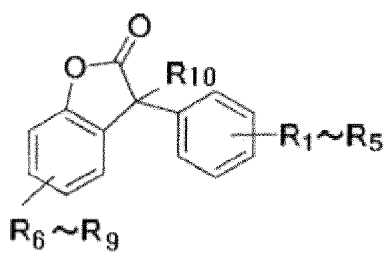
(1)

(in General formula (1),

$R_1$-$R_5$ each independently represent a hydrogen atom or an optionally substituted alkyl group with a total of 1-20 carbon atoms,

$R_6$-$R_9$ each independently represent a hydrogen atom or an optionally substituted alkyl group with a total of

1-20 carbon atoms, and
$R_{10}$ represents a hydrogen atom or an alkyl group with a total of 1-5 carbon atoms.)

<31> A method for improving the transmissivity of a thermoplastic resin composition, the method comprising a step of adding a compounding agent represented by General formula (1) below to a thermoplastic resin:

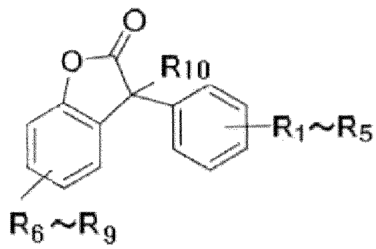

(1)

(in General formula (1),

$R_1$-$R_5$ each independently represent a hydrogen atom or an optionally substituted alkyl group with a total of 1-20 carbon atoms,
$R_6$-$R_9$ each independently represent a hydrogen atom or an optionally substituted alkyl group with a total of 1-20 carbon atoms, and
$R_{10}$ represents a hydrogen atom or an alkyl group with a total of 1-5 carbon atoms.)

<32> A method for lowering the haze of a thermoplastic resin composition, the method comprising a step of adding a compounding agent represented by General formula (1) below to a thermoplastic resin:

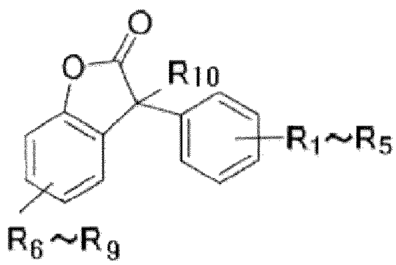

(1)

(in General formula (1),

$R_1$-$R_5$ each independently represent a hydrogen atom or an optionally substituted alkyl group with a total of 1-20 carbon atoms,
$R_6$-$R_9$ each independently represent a hydrogen atom or an optionally substituted alkyl group with a total of 1-20 carbon atoms, and
$R_{10}$ represents a hydrogen atom or an alkyl group with a total of 1-5 carbon atoms.)

ADVANTAGEOUS EFFECT OF THE INVENTION

As described above, a thermoplastic resin composition of the present invention contains a certain compounding agent to maintain transmissivity, especially in the low wavelength range, at a good level. For example, whereas the addition of an antioxidant and mold release agent was found to have a tendency of decreasing the transmissivity in a conventional thermoplastic resin composition, a decrease in transmissivity, especially in the low-wavelength range, can be prevented in a thermoplastic resin composition of the present invention even when such additives are added. Such thermoplastic resin composition is particularly suitable as an optical material.

BRIEF DESCRIPTION OF DRAWINGS

Figure 1: A graph showing the powder X-ray diffraction pattern of a crystal of a compounding agent used in Example 1.
Figure 2: A graph showing the powder X-ray diffraction pattern of a crystal of the same compound as the compounding agent in Example 1, but for a different sample.
Figure 3: A graph showing the powder X-ray diffraction pattern of a crystal of the same compound as the compounding agent in Example 1, but for another different sample.

EMBODIMENTS FOR CARRYING OUT THE INVENTION

[1. Components of thermoplastic resin composition]

Hereinafter, components of a thermoplastic resin composition will be described.

[1-1. Compounding agent]

A thermoplastic resin composition comprises a compounding agent represented by General formula (1) below. The compounding agent represented by Formula (1) is used to improve the value of transmissivity of the thermoplastic resin composition, especially at a low wavelength.
The addition of the compounding agent represented by General formula (1) can also lower the haze and improve the transparency of the thermoplastic resin composition.

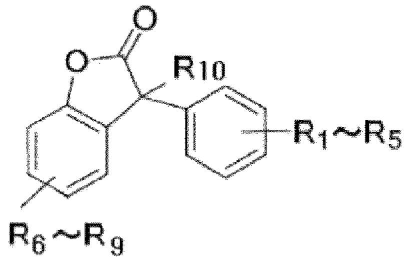

(1)

In General formula (1), $R_1$-$R_5$ each independently represent a hydrogen atom or an optionally substituted alkyl group with a total of 1-20 carbon atoms. $R_1$-$R_5$ are preferably a hydrogen atom or an optionally substituted alkyl group with a total of 1-10 carbon atoms, where the total number of the carbon atoms in the optionally substituted alkyl group is preferably 1-5 and more preferably 1-3, and the alkyl group is, for example, a methyl group.
Moreover, as to $R_1$-$R_5$ in General formula (1), preferably, two to four of them are hydrogen atoms while one to three of them are alkyl groups, and more preferably, three of them are hydrogen atoms while two of them are alkyl groups.
In General formula (1), $R_6$-$R_9$ each independently represent a hydrogen atom or an optionally substituted alkyl group with a total of 1-20 carbon atoms. $R_6$-$R_9$ are preferably a hydrogen atom or an optionally substituted alkyl group with a total of 1-10 carbon atoms, where the total number of the carbon atoms in the optionally substituted alkyl group is preferably 1-8 and more preferably 1-5, and the alkyl group is, for example, a t-butyl group.
Moreover, as to $R_6$-$R_9$ in General formula (1), preferably, one to three of them are hydrogen atoms and one to three of them are alkyl groups, and more preferably, two of them are hydrogen atoms while two of them are alkyl groups.
In General formula (1), $R_{10}$ represents a hydrogen atom or an alkyl group with a total of 1-5 carbon atoms. $R_{10}$ is preferably a hydrogen atom or an optionally substituted alkyl group with a total of 1-3 carbon atoms, where the total number of the carbon atoms in the optionally substituted alkyl group is preferably 1 or 2. More preferably, $R_{10}$ is a hydrogen atom.
While the carbon atom bonded to $R_{10}$ is a chiral carbon in General formula (1), the compounding agent in General formula (1) may be racemic or optically active.
The above-mentioned substituent in General formula (1) is, for example, any of a halogen, a cyano group, an alkenyl group, an alkynyl group, and an alkoxy group.
Specific examples of the compounding agent represented by General formula (1) include compounds represented by Formulae (10) and (11) below, and a mixture thereof.

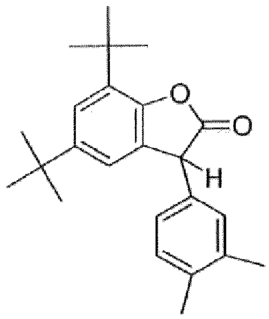

(1 0)

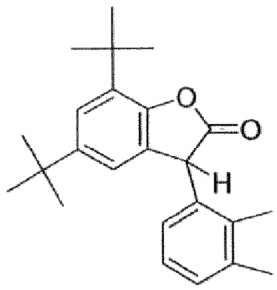

(1 1)

A thermoplastic resin composition may contain the compounding agent in an amount of 1-10,000 ppm by weight. The content of the compounding agent may be 1-8,000 ppm by weight, 1-6,000 ppm by weight, 1-4,000 ppm by weight, or 1-3,000 ppm by weight.

In the thermoplastic resin composition, the compounding agent is preferably contained in an amount of 1-2,000 ppm by weight based on the total weight of the thermoplastic resin composition. The content of the compounding agent in the thermoplastic resin composition is more preferably 10-1,000 ppm by weight, still more preferably 50-800 ppm by weight, yet still more preferably 50-500 ppm by weight, and even more preferably 100-300 ppm by weight.

The compounding agent is preferably crystalline. For example, a crystal of a 90:10 mixture of the compounds of Formulae (10) and (11) above (i.e., in General formula (1), two of $R_1$-$R_5$ are methyl groups while the other two are hydrogen, and two of $R_6$-$R_9$ are tertiary butyl groups while the other two are hydrogen) has peaks at diffraction angles 2θ of 6.7°, 10.4°, 11.1°, 12.7°, 13.2°, 15.2°, 16.1°, 17.3°, 20.8°, and 23.6° in a powder X-ray diffraction pattern using Cu-Kα radiation under the measurement conditions described in the section under EXAMPLES. Among others, the intensities of the peaks at 13.2°, 15.2° and 20.8° were relatively high.

As shown in the section under EXAMPLES, we determined the peaks of the powder X-ray diffraction patterns using Cu-Kα radiation for multiple samples of the compounding agent having the same compound, and confirmed that all samples had the above peak values almost in common. However, a measurement error of about ±0.2° or ±0.1° may occur. Therefore, the above-described compounding agent has peaks at diffraction angles 2θ of 6.7 ± 0.2°, 10.4 ± 0.2°, 11.1 ± 0.2°, 12.7 ± 0.2°, 13.2 ± 0.2°, 15.2 ± 0.2°, 16.1 ± 0.2°, 17.3 ± 0.2°, 20.8 ± 0.2°, and 23.6 ± 0.2° in a powder X-ray diffraction pattern using Cu-Kα radiation.

In addition to the above-described compounding agent, the thermoplastic resin composition may further contain the following additives.

[1-2. Antioxidant]

The thermoplastic resin composition preferably contains an antioxidant.

The antioxidant is preferably at least either a phenolic antioxidant or a phosphite-based antioxidant. Furthermore, phenolic and phosphite-based antioxidants may be used in combination, and a thermoplastic resin composition containing both phenolic and phosphite-based antioxidant is preferable.

Examples of the phenolic antioxidant include 1,3,5-tris(3,5-di-tert-butyl-4-hydroxyphenylmethyl)-2,4,6-trimethylbenzene, 1,3,5-tris(3,5-di-tert-butyl-4-hydroxybenzyl)-1,3,5-triazine-2,4,6(1H,3H,5H)-trione, 4,4',4"-(1-methylpropanyl-3-ylidene)tris(6-tert-butyl-m-cresol), 6,6'-di-tert-butyl-4,4'-butylidenedi-m-cresol, octadecyl 3-(3,5-di-tert-butyl-4-hydroxyphenyl)propionate, pentaerythritol-tetrakis[3-(3,5-di-tert-butyl-4-hydroxyphenyl)propionate], 3,9-bis{2-[3-(3-tert-butyl-4-hydroxy-5-methylphenyl)propionyloxy]-1,1-dimethylethyl}-2, 4,8,10-tetraoxospiro[5.5]undecane, and pentaerythritol-tetrakis[3-(3,5-di-tert-butyl-4-hydroxyphenyl)propionate], where pentaerythritol-tetrakis[3-(3,5-di-tert-butyl-4-hydroxyphenyl)propionate] is preferable.

Examples of the phosphite-based antioxidant include 2-ethylhexyl diphenyl phosphite, isodecyldiphenyl phosphite, triisodecyl phosphite, triphenyl phosphite, 3,9-bis(octadecyloxy)-2,4,8,10-tetraoxy-3,9-diphosphaspiro[5.5]undecane, 3,9-bis(2,6-di-tert-butyl-4-methylphenoxy)-2,4,8,10-tetraoxa-3,9-diphosphaspiro[5.5]unde cane, 2,2'-methylenebis(4,6-di-tert-butylphenyl-2-ethylhexyl phosphite, tris(2,4-di-tert-butylphenyl)phosphite, tris(nonylphenyl)phosphite, tetra-C12-15-alkyl (propane-2,2-diylbis(4,1-phenylene))bis(phosphite), and 3,9-bis(2,6-di-tert-butyl-4-methylphenoxy)-2,4,8,10-tetraoxa-3,9-diphosphaspiro[5.5]unde cane, where 3,9-bis(2,6-di-tert-butyl-4-methylphenoxy)-2,4,8,10-tetraoxa-3,9-diphosphaspiro[5.5]unde cane is preferable.

As the antioxidant, one of the above-mentioned antioxidants may be used alone, or a mixture of two or more kinds of them may be used.

A thermoplastic resin composition may contain the antioxidant in an amount of 1-10,000 ppm by weight. The content of the antioxidant may be 1-8,000 ppm by weight, 1-6,000 ppm by weight, or 1-4,000 ppm by weight.

In the thermoplastic resin composition, the antioxidant is preferably contained in an amount of 1-3,000 ppm by weight based on the total weight of the resin composition. The content of the antioxidant in the thermoplastic resin composition is more preferably 50-2,500 ppm by weight, still more preferably 100-2,000 ppm by weight, yet still more preferably 150-1,500 ppm by weight, and even more preferably 200-1,200 ppm by weight.

While the above-mentioned ranges of the antioxidant content refer to the content of the antioxidants in total, the above-mentioned range may also be applied to the content of any one kind of antioxidant.

[1-3. Mold release agent]

The thermoplastic resin composition preferably contains a mold release agent.

Examples of the mold release agent include an ester compound, for example, a glycerol fatty acid ester such as a mono- or di-glyceride of a glycerol fatty acid, a glycol fatty acid ester such as a propylene glycol fatty acid ester and a sorbitan fatty acid ester, a higher alcohol fatty acid ester, and a full ester or mono-fatty acid ester of an aliphatic polyhydric alcohol and an aliphatic carboxylic acid. When an ester of an aliphatic polyhydric alcohol and an aliphatic carboxylic acid is used as the mold release agent, it may be a monoester, full ester, or any other ester, for example, it may not be a full ester (e.g., a monoester).

Specific examples of the mold release agent include the following:

a sorbitan fatty acid ester such as sorbitan stearate, sorbitan laurate, sorbitan oleate, sorbitan trioleate, sorbitan tribehenate, sorbitan stearate, sorbitan tristearate, and sorbitan caprylate;
a propylene glycol fatty acid ester such as propylene glycol monostearate, propylene glycol monooleate, propylene glycol monobehenate, propylene glycol monolaurate, and propylene glycol monopalmitate;
a higher alcohol fatty acid ester such as stearyl stearate;
a monoglyceride of a glycerol fatty acid ester, including: a monoglyceride of glycerol monostearate, glycerol monohydroxystearate (e.g., glycerol mono-12-hydroxy stearate), glycerol monooleate, glycerol monobehenate, glycerol monocaprylate, glycerol monocaprate, and glycerol monolaurate: and a mono/di-glyceride of glycerol mono/distearate, glycerol mono/di-stearate, glycerol mono/di-behenate, glycerol mono/di-diolate, etc.;
an acetylated monoglyceride of a glycerol fatty acid ester such as glycerol diacetomonolaurate;
an organic acid monoglyceride of a glycerol fatty acid ester such as monoglyceride of citric acid fatty acid, monoglyceride of succinic acid fatty acid, and monoglyceride of diacetyl tartaric acid fatty acid; and
a polyglycerol fatty acid ester such as diglycerol stearate, diglycerol laurate, diglycerol oleate, diglycerol monostearate, diglycerol monolaurate, diglycerol monomyristate, diglycerol monooleate, tetraglycerol stearate, decaglycerol laurate, decaglycerol oleate, and polyglycerol polyricinoleate.

The mold release agent is contained in the thermoplastic resin composition preferably in an amount of 1-5,000 ppm by weight based on the total weight of the resin composition. The content of the mold release agent in the thermoplastic resin composition is more preferably 50-4,000 ppm by weight, still more preferably 100-3,500 ppm by weight, yet still more preferably 500-13,000 ppm by weight, and even more preferably 1,000-2,500 ppm by weight.

The mold release agent can be used in combination with other additive such as an antioxidant. Although the above-mentioned content of the mold release agent refers to the content of the mold release agents in total, the above-mentioned range may also refer to the content of one kind of mold release agent.

[1-4. Catalyst deactivator]

Preferably, the thermoplastic resin composition further contains a catalyst deactivator as an additive. A catalyst deactivator terminates polymerization reaction by causing the catalyst for the polymerization of the resin composition to lose its activity. The addition of the catalyst deactivator can also prevent the depolymerization of the polymer contained

in the resin composition. Alternatively, the catalyst deactivator may not be used in order to prevent an increase in the thermal history of the resin composition, which is caused by the addition of the catalyst deactivator.

As the catalyst deactivator, an ester such as butyl benzoate; an aromatic sulfonic acid such as p-toluenesulfonic acid; an aromatic sulfonic ester such as butyl p-toluenesulfonate and hexyl p-toluenesulfonate; a phosphoric acid such as phosphorous acid, phosphoric acid, and phosphonic acid; a phosphite ester such as triphenyl phosphite, monophenyl phosphite, diphenyl phosphite, diethyl phosphite, di-n-propyl phosphite, di-n-butyl phosphite, di-n-hexyl phosphite, dioctyl phosphite, and monooctyl phosphite; a phosphoric ester such as triphenyl phosphate, diphenyl phosphate, monophenyl phosphate, dibutyl phosphate, dioctyl phosphate, and monooctyl phosphate; a phosphonic acid such as diphenyl phosphonic acid, dioctyl phosphonic acid, and dibutyl phosphonic acid; a phosphonic ester such as diethyl phenyl phosphonic acid; a phosphine such as triphenyl phosphine and bis(diphenylphosphino)ethane; a boric acid such as boric acid and phenylboric acid; an aromatic sulfonate such as tetrabutylphosphonium salt of dodecylbenzene sulfonate; an organic halide such as stearic acid chloride, benzoyl chloride, and p-toluenesulfonate chloride; an alkyl sulfate such as dimethyl sulfate; and organic halide such as benzyl chloride can suitably be used. In terms of the effect of the deactivator, stability against the resin, and the like, tetrabutylphosphonium salt of dodecylbenzene sulfonate, p-toluene, or butyl sulfonate is particularly preferred. The deactivator is used in an amount that is 0.01-50 times, preferably 0.3-20 times the molar amount of the catalyst. If the amount is less than 0.01 times the molar amount of the catalyst, the deactivation effect will be inadequate, which is unfavorable. On the other hand, if the amount is greater than 50 times the molar amount of the catalyst, the heat resistance of the resin will decrease and the molded article will easily be colored, which are undesirable.

In thermoplastic resin composition, the catalyst deactivator is preferably contained in an amount of 1-1,000 ppm by weight based on the total weight of the resin composition. The content of the catalyst deactivator in the thermoplastic resin composition is more preferably 3-500 ppm by weight, still more preferably 5-100 ppm by weight, and particularly preferably 10-50 ppm by weight.

The catalyst deactivator may be added to the thermoplastic resin composition, preferably as a solution, for example, as an aqueous solution. The catalyst deactivator may be added to the thermoplastic resin composition as a solution in an alcohol such as methanol or ethanol, or as a solution in an organic solvent such as a phenol solution.

[1-5. Other additives]

In addition to the above-described compounding agent, antioxidant, mold release agent, and catalyst deactivator, other additives may be added to the thermoplastic resin composition. For example, examples of the additives that may be contained in the thermoplastic resin composition include a heat stabilizer, a plasticizer, a filler, a UV absorber, an anti-rust agent, a dispersant, a defoaming agent, a leveling agent, a flame retardant, a lubricant, a dye, a pigment, a bluing agent, a nucleating agent, and a clarifying agent.

The content of additives (hereinafter also referred to as "optional additives") other than the compounding agent, antioxidant, mold release agent, and catalyst deactivator in the thermoplastic resin composition is preferably, but is not limited to, 10 ppm to 5.0% by weight, more preferably 100 ppm to 2.0% by weight, and still more preferably 1,000 ppm to 1.0% by weight.

The additives mentioned above may adversely affect transmissivity and thus are preferably not added in excess, and the total amount added is, for example, within the range mentioned above.

[1-6. Thermoplastic resin]

The thermoplastic resin composition contains a thermoplastic resin.

The thermoplastic resin is preferably any one or more of a polycarbonate resin, a polyester resin, a polyester carbonate resin, a cyclo-olefin-based resin, and an acrylic resin.

The thermoplastic resin preferably contains a polycarbonate resin, a polyester resin, or a polyester carbonate resin, which has a structural unit (B) derived from a monomer represented by General formula (2) below.

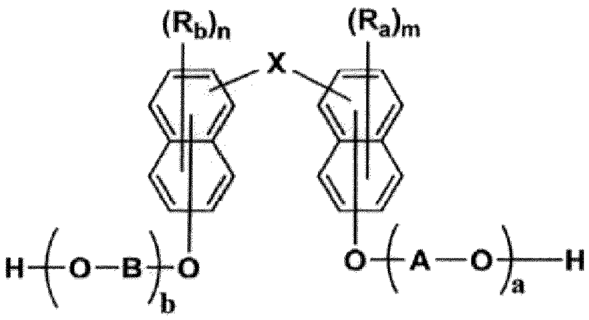

(2)

In General formula (2), $R_a$ and $R_b$ are each independently selected from the group consisting of a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 6-20 carbon atoms containing one or more heteroatoms selected from O, N and S, an optionally substituted aryloxy group with 6-20 carbons, and $-C\equiv C-R_h$. $R_h$ represents an optionally substituted aryl group with 6-20 carbon atoms or an optionally substituted heteroaryl group with 6-20 carbon atoms containing one or more heteroatoms selected from O, N and S.

$R_a$ and $R_b$ are preferably a hydrogen atom, an optionally substituted aryl group with 6-20 carbon atoms, or an optionally substituted heteroaryl group with 6-20 carbon atoms containing one or more heteroatoms selected from O, N and S. They are more preferably hydrogen atoms or optionally substituted aryl groups with 6-20 carbon atoms, and still more preferably hydrogen atoms or optionally substituted aryl groups with 6-12 carbon atoms.

In General formula (2), X represents a single bond or an optionally substituted fluorene group. X is preferably a single bond, or an optionally substituted fluorene group with a total of 12-20 carbon atoms.

In General formula (2), A and B are each independently an optionally substituted alkylene group with 1-5 carbon atoms, and preferably an alkylene group with 2 or 3 carbon atoms.

In General formula (2), m and n are each independently an integer from 0 to 6, preferably an integer from 0 to 3, and more preferably 0 or 1.

In General formula (2), a and b are each independently an integer from 0 to 10, preferably an integer from 1 to 3, and more preferably 1 or 2.

Specific examples of the structural unit (B) include those derived from BNE, DPBHBNA, or the like.

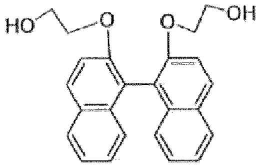

(BNE)

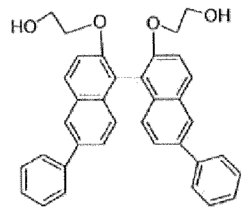

(DPBHBNA)

The thermoplastic resin preferably contains a polycarbonate resin, a polyester resin, or a polyester carbonate resin, which has a structural unit (C) derived from a monomer represented by General formula (3) below.

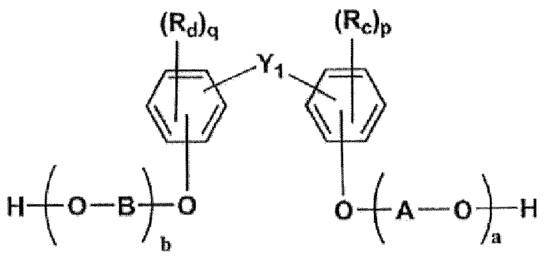

(3)

In General formula (3), $R_c$ and $R_d$ are each independently selected from the group consisting of a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl

group with 5-20 carbon atoms, and an optionally substituted aryl group with 6-20 carbon atoms.

$R_c$ and $R_d$ are preferably a hydrogen atom, an optionally substituted aryl group with 6-20 carbon atoms, or an optionally substituted heteroaryl group with 6-20 carbon atoms containing one or more heteroatoms selected from O, N and S. They are more preferably a hydrogen atom or an optionally substituted aryl group with 6-20 carbon atoms, and still more preferably a hydrogen atom or an optionally substituted aryl group with 6-12 carbon atoms.

In General formula (3), $Y_1$ is a single bond, an optionally substituted fluorene group, or any of the structural formulae represented by General formulae (4) to (9) and (12) to (14) below. Preferably, it is a single bond or is represented by a structural formula represented by General formula (4) below.

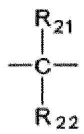

(4)

$-S-$

(5)

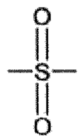

(6)

$-(CH_2)_r-$

(7)

$-O-$

(8)

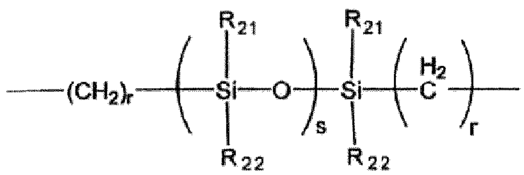

(9)

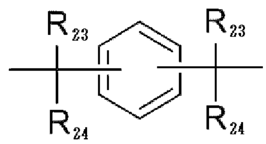

(12)

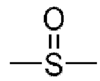

(13)

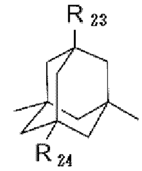

(14)

In General formulae (4) and (9), $R_{21}$ and $R_{22}$ each independently represent a hydrogen atom, a halogen atom, an optionally substituted alkyl group with 1-20 carbon atoms, an optionally substituted aryl group with 6-30 carbon atoms, or an optionally substituted carbon or heterocyclic ring with 1-20 carbon atoms which is formed of $R_{21}$ and $R_{22}$ bonding to each other.

In General formulae (7) and (9), r and s each independently represent an integer from 0 to 5,000;

Furthermore, in General formulae (12) to (14), $R_{23}$ and $R_{24}$ are each independently a hydrogen atom, fluorine, chlorine, bromine, iodine, an optionally substituted alkyl group with 1-9 carbon atoms, an optionally substituted alkoxy group with 1-5 carbon atoms, an optionally substituted alkenyl group with 2-12 carbon atoms, or an optionally substituted aryl group with 6-12 carbon atoms.

In General formula (3) above, A and B are each independently an optionally substituted alkylene group with 1-5 carbon atoms, and preferably an alkylene group with 2 or 3 carbon atoms. In General formula (3), p and q are each independently an integer from 0 to 4, and preferably 0 or 1. In addition, in General formula (3) above, a and b are each independently an integer from 0 to 10, preferably an integer from 0 to 5, more preferably an integer from 0 to 2, and, for example, 0 or 1.

Specific examples of the structural unit (C) include those derived from BPEF (9,9-bis(4-(2-hydroxyethoxy)phenyl)fluorene), BPPEF (9,9-bis(4-(2-hydroxyethoxy)-3-phenylphenyl)fluorene), bisphenol A, bisphenol AP, bisphenol AF, bisphenol B, bisphenol BP, bisphenol C, bis(4-hydroxyphenyl)-2,2-dichloroethylene, bisphenol E, bisphenol F, bisphenol G, bisphenol M, bisphenol S, bisphenol P, bisphenol PH, bisphenol TMC, bisphenol P-AP (4,4'-(1-phenylethylidene)bisphenol), bisphenol P-CDE (4,4'-cyclododecylidenebisphenol), bisphenol P-HTG (4,4'-(3,3,5-trimethylcyclohexylidene)bisphenol)), bisphenol P-MIBK (4,4'-(1,3-dimethylbutylidene)bisphenol), bisphenol PEO-FL (bisphenoxyethanol fluorene), bisphenol P-3MZ (4-[1-(4-hydroxyphenyl)-3-methylcyclohexyl]phenol), bisphenol OC-FL (4,4'-[1-[4-[1-(4-hydroxyphenyl)-1-methylethyl]phenyl]ethylidene]bisphenol), bisphenol Z, BP-2EO (2,2'-[[1,1'-biphenyl]-4,4'-diylbis(oxy)bis-ethanol), S-BOC (4,4'-(1-methylethylidene)bis(2-methylphenol), and TrisP-HAP (4,4',4"-ethylidene trisphenol).

Thermoplastic resins such as polycarbonate resins that have a structural unit derived from a bisphenol compound such as bisphenol A or bisphenol AP as the structural unit (C) are advantageous in that they are often of high purity and their market availability is good.

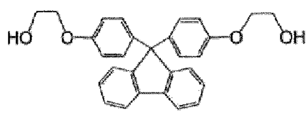

(BPEF)

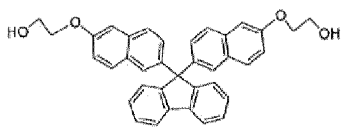

(BNEF)

The thermoplastic resin may be a polymer which contains the structural unit (B) but not the structural unit (C), a polymer which contains the structural unit (C) but not the structural unit (B), a copolymer having the structural units (B) and (C), a mixture of a polymer having the structural unit (B) alone and a polymer having the structural unit (C) alone, or a combination thereof. Examples of the polymer that contains the structural unit (C) but not the structural unit (B) include those having structural units represented by Formulae (I-1) to (I-3) below, while examples of the copolymer having the structural units (B) and (C) include those having structural units represented by Formulae (II-1) to (II-4) below. Specific examples of the polymer that contains the structural unit (C) but not the structural unit (B) include those consisting of one or multiple kinds of the above-mentioned BPEF, BPPEF, and bisphenols.

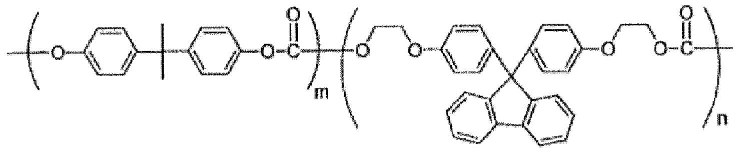

(I-1)

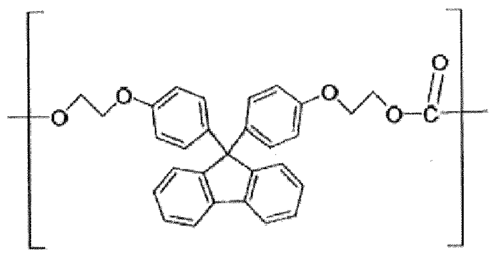

(I-2)

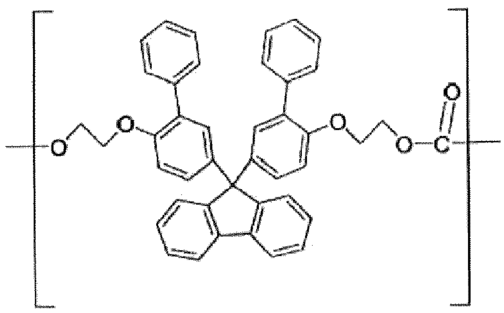

(I-3)

(In formula (I-1), m and n are each an integer from 1 to 10, preferably an integer from 1 to 5, and more preferably 1, and in formula (I-3), n is an integer from 1 to 10, preferably an integer from 1 to 5, and more preferably 1.)

While either a block copolymer in which m and n are high (e.g., 100 or higher) or a random copolymer can be used as the polymer having multiple kinds of structural units, it is preferable to use a random copolymer, and it is more preferable to use a random copolymer in which m and n are 1.

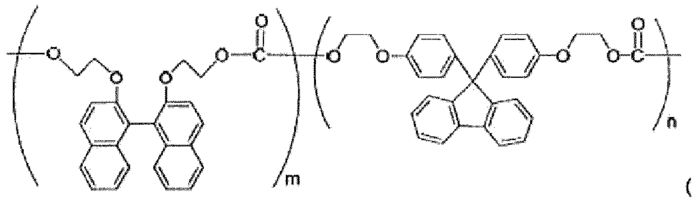

(II-1)

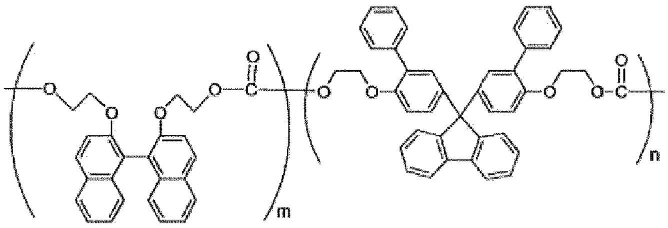

(II-2)

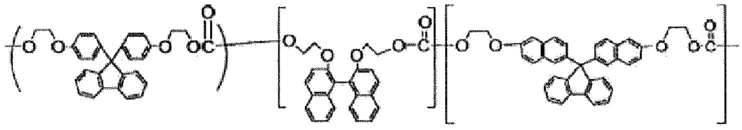

(II-3)

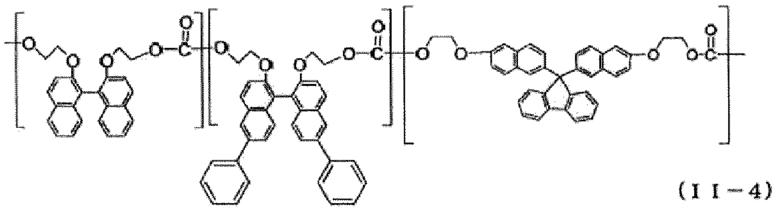

(II-4)

(In Formulae (II-1) to (II-4), m and n are each independently an integer from 1 to 10, preferably an integer from 1 to 5, and more preferably 1.)

Although either a block copolymer in which m and n are high (e.g., 100 or higher) or a random copolymer can be used as the polymer having multiple kinds of structural units, it is preferable to use a random copolymer, and it is more preferable to use a random copolymer in which m and n are 1.

In the above copolymer, the mole ratio of the structural unit (B) to the structural unit (C) is preferably 1:99-99:1, more preferably 10:90-90:10, still more preferably 15:85-85:15, and particularly preferably 30:70-70:30. In the above mixture, the weight ratio of the polymer having the structural unit (B) alone to the polymer having the structural unit (C) alone is preferably 1:99-99:1, more preferably 10:90-90:10, still more preferably 15:85-85:15, and particularly preferably 30:70-70:30.

The thermoplastic resin may be a cyclo-olefin-based resin or may contain a cyclo-olefin-based resin. Examples of the cyclo-olefin-based resin include those having the following structural units.

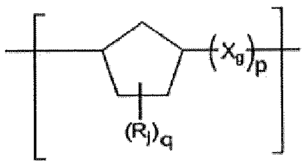

(20)

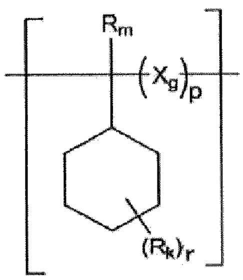

(21)

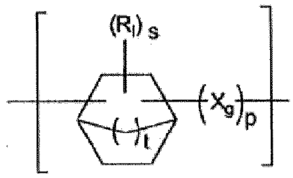

(2 2)

In the above formula, $X_g$ each independently represent an alkylene group with 1-10 carbon atoms. Examples of the alkylene group with 1-10 carbon atoms include methylene, ethylene, propylene, isopropylene, butylene, isobutylene, sec-butylene, tert-butylene, and pentylene. Among them, methylene, ethylene, propylene, butylene, isobutylene, and sec-butylene are preferable, and methylene, ethylene, and propylene are more preferable.

$R_j$, $R_k$, and $R_l$ are each independently selected from a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 3-20 carbon atoms containing one or more heteroatoms selected from O, N and S, an optionally substituted aryloxy group with 6-20 carbon atoms, and -C≡C-$R_i$, Examples of $R_j$, $R_k$, and $R_l$ above are the same as those listed for $X_a$, $X_b$, $X_c$, $X_a$, $X_e$, and $X_f$ above.

However, $R_j$, $R_k$, and $R_l$ may have a substituent. Examples of such a substituent include, but not limited to, a halogen atom, an alkyl group with 1-10 carbon atoms, a cycloalkyl group with 5-10 carbon atoms, an alkoxy group with 1-10 carbon atoms, a cycloalkyloxy group with 5-10 carbon atoms, an alkyloxycarbonyl group with 2-10 carbon atoms, a cycloalkyloxycarbonyl group with 5-10 carbon atoms, an aryloxycarbonyl group with 7-15 carbon atoms, an alkylcarbonyloxy group with 2-10 atoms, a cycloalkylcarbonyloxy group with 5-10 carbon atoms, an aryl carbonyloxy group with 7-15 carbon atoms, a hydroxyalkylcarbonyl group with 2-10 carbon atoms, a glycidyloxycarbonyl group, a hydroxy group, a carboxy group, a cyano group, and an amide group with 1-10 carbon atoms.

Examples of the above alkyl group with 1-10 carbon atoms include a methyl group, an ethyl group, a propyl group, an isopropyl group, a butyl group, an isobutyl group, a sec-butyl group, a tert-butyl group, and a pentyl group..

Examples of the above cycloalkyl group with 5-10 carbon atoms include a cyclopentyl group, a cyclohexyl group, a cycloheptyl group, a bicyclo[2.2.1]heptyl group, and a bicyclo[2.2.2]octyl group.

Examples of the above alkoxy group with 1-10 carbon atoms include a methoxy group, an ethoxy group, a propyloxy group, an isopropyloxy group, a butoxy group, an isobutoxy group, a sec-butoxy group, a tert-butoxy group, and a pentyloxy group.

Examples of the above cycloalkyloxy group with 5-10 carbon atoms include a cyclopentyloxy group, a cyclohexyloxy group, a bicyclo[2.2. 1]heptyloxy group, and a bicyclo[2.2.2]octyloxy group.

Examples of the above alkyloxycarbonyl group with 2-10 carbon atoms include a methyloxycarbonyl group, an ethyloxycarbonyl group, a propyloxycarbonyl group, an isopropyloxycarbonyl group, a butyloxycarbonyl group, an isobutyloxycarbonyl group, a sec-butyloxycarbonyl group, and a tert-butyloxycarbonyl group.

Examples of the above cycloalkyloxycarbonyl group with 5-10 carbon atoms include a cyclopentyloxycarbonyl group, a cyclohexyloxycarbonyl group, a bicyclo[2.2.1]heptyloxycarbonyl group, and a bicyclo[2.2.2]octyloxycarbonyl group.

Examples of the aryloxycarbonyl group with 7-15 carbon atoms include a phenyloxycarbonyl group, a tolyloxycarbonyl group, a xylyloxycarbonyl group, a trimethylphenyloxycarbonyl group, a tetramethylphenyloxycarbonyl group, an ethylphenyloxycarbonyl group, an ethylmethylphenyloxycarbonyl group, a diethylphenyloxycarbonyl group, and a naphthyloxycarbonyl group.

Examples of the above alkylcarbonyloxy group with 2-10 carbon atoms include a methylcarbonyloxy group, an ethylcarbonyloxy group, a propylcarbonyloxy group, an isopropylcarbonyloxy group, and a butylcarbonyloxy group.

Examples of the above cycloalkylcarbonyloxy group with 5-10 carbons include a cyclopentylcarbonyloxy group, a cyclohexylcarbonyloxy group, a bicyclo[2.2.1]heptylcarbonyloxy group, and a bicyclo[2.2.2]octylcarbonyloxy group.

Examples of the above aryl carbonyloxy group with 7-15 carbon atoms include a phenylcarbonyloxy group, a tolylcarbonyloxy group, a xylylcarbonyloxy group, a trimethylphenylcarbonyloxy group, a tetramethylphenylcarbonyloxy group, an ethylphenylcarbonyloxy group, an ethylmethylphenylcarbonyloxy group, a diethylphenylcarbonyloxy group, and a naphthylcarbonyloxy group.

Examples of the above hydroxyalkyl carbonyl group with 2-10 carbon atoms include a hydroxymethylcarbonyl group, a hydroxyethylcarbonyl group, and a hydroxypropylcarbonyl group.

Examples of the above amide group with 1-10 carbon atoms include a methylaminocarbonyl group, an ethylaminocarbonyl group, a dimethylaminocarbonyl group, and an acetylamino group.

The above substituents may be contained alone or in combination of two or more.

$R_i$ represents an aryl group with 6-20 carbon atoms or a heteroaryl group with 3-20 carbon atoms containing one or more heteroatoms selected from O, N and S. $R_i$ above is the same as described above.

p each independently represent an integer of 0 or 1.

q, r, and s are each independently an integer from 0 to 10, preferably 0 to 5, and more preferably 0 to 3.

t represents an integer from 1 to 3, and is preferably 1 or 2.

When q is greater than or equal to 2 and two $R_j$ are present on adjacent carbon atoms, the two $R_j$ may form a ring structure with each other. For example, if q is 2 and the two $R_j$ are both optionally substituted alkyl groups, then General formula (20) is represented by Formula (20-1) below, and if q is 2 and the two $R_j$ are an optionally substituted alkyl group and an optionally substituted cycloalkyl group, respectively, then General formula (20) is represented by (20-2), (20-3), or (20-4) below.

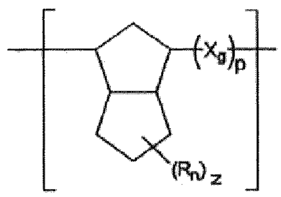
(20-1)

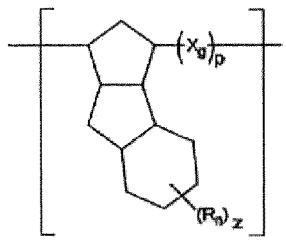
(20-2)

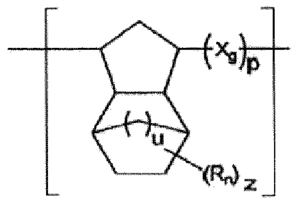
(20-3)

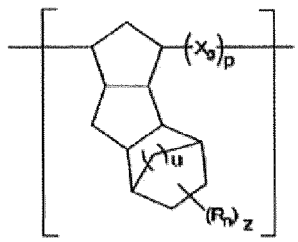
(20-4)

In the above formulae, $X_g$ and p are as described above.

$R_n$ represents the above-mentioned substituent, where specific examples thereof include a halogen atom, an alkyl group with 1-10 carbon atoms, a cycloalkyl group with 5-10 carbon atoms, an alkoxy group with 1-10 carbon atoms, a cycloalkyloxy group with 5-10 carbon atoms, an alkyloxycarbonyl group with 2-10 carbon atoms, a cycloalkyloxycarbonyl group with 5-10 carbon atoms, an aryloxycarbonyl group with 7-15 carbon atoms, an alkylcarbonyloxy group with 2-10 atoms, a cycloalkylcarbonyloxy group with 5-10 carbon atoms, an aryl carbonyloxy group with 7-15 carbon atoms, a hydroxyalkylcarbonyl group with 2-10 carbon atoms, a glycidyloxycarbonyl group, a hydroxy group, a carboxy group, a cyano group, and an amide group with 1-10 carbon atoms.

While z is not particularly limited, it is preferably 0-6, more preferably 0-3, and still more preferably 0 or 1.

u represents an integer from 1 to 3, and is preferably 1 or 2.

In addition, when r is greater than or equal to 2 and two $R_k$ are present on adjacent carbon atoms, the two $R_k$ may form a ring structure with each other. For example, if r is 2 and the two $R_k$ are both optionally substituted alkyl groups, then General formula (21) is represented by Formula (21-1) or (21-2) below, and if r is 2 and the two $R_k$ are an optionally substituted alkyl group and an optionally substituted cycloalkyl group, respectively, then General formula (21) is represented by (21-3) below.

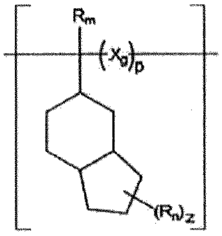

(21-1)

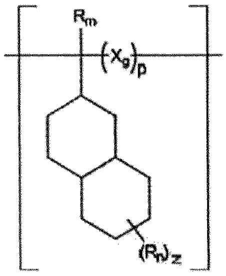

(21-2)

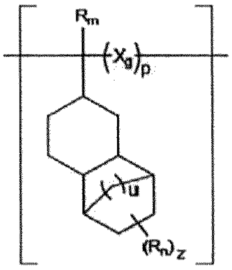

(21-3)

In the above formula, $X_g$, p, $R_n$, z, and u are as described above.

Furthermore, when s is greater than or equal to 2 and two $R_l$ are present on adjacent carbon atoms, the two $R_l$ may form a ring structure with each other. For example, if s is 2 and the two $R_l$ are both optionally substituted alkyl groups, then General formula (22) is represented by (22-1) or (22-2) below, and if s is 2 and the two $R_l$ are an optionally substituted alkyl group and an optionally substituted cycloalkyl group, respectively, then General formula (22) is represented by (22-3) or (22-4) below.

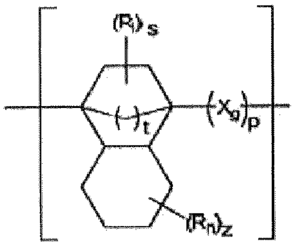

(22-1)

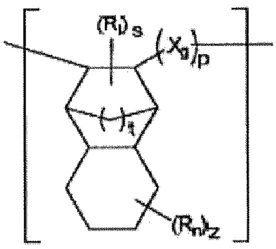

(22-2)

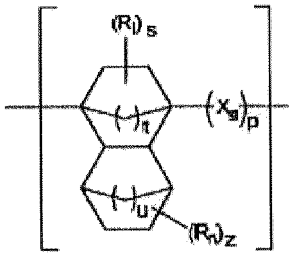

(2 2 − 3)

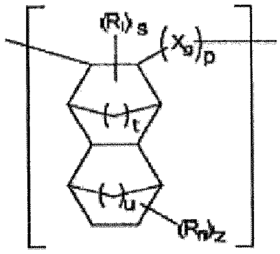

(2 2 − 4)

In the above formula, $X_g$, p, $R_n$, z, and u are as described above.

$R_m$ represents a hydrogen atom or an alkyl group with 1-3 carbon atoms. Examples of the alkyl group with 1-3 carbon atoms include, but are not limited to, a methyl group, an ethyl group, a propyl group, and an isopropyl group.

Specific examples of the cyclo-olefin-based resin include those containing at least one selected from the group consisting of the structural units represented by Formulae 1-8 below.

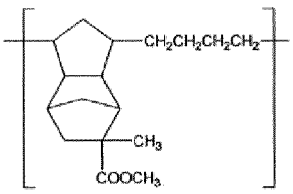

1

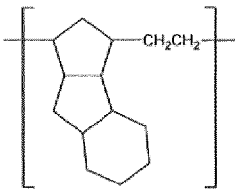

2

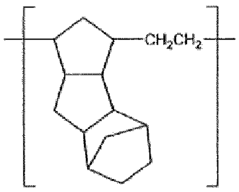

3

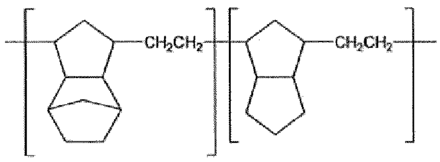

4

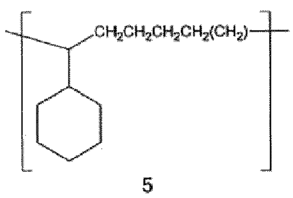

5

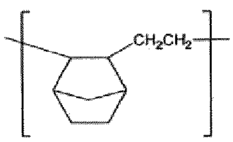

6

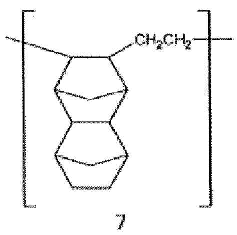

7

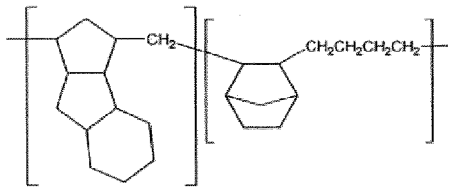

8

The above-mentioned structural units may be contained in the cyclo-olefin-based resin alone or in a combination of two or more. In addition, the above-mentioned structural units may be combined with a structural unit of other cyclic polyolefin, or with a structural unit of other resin (polyolefin resin, polyester resin) or the like.

While the weight-average molecular weight (Mw) of the cyclo-olefin-based resin is not particularly limited, it is preferably 1,000-3,000,000, more preferably 10,000-3,000,000, still more preferably 20,000-1,000,000, and particularly preferably 30,000-500,000.

Other than the above-mentioned cyclo-olefin-based resin, a resin (polymer) having a structural unit containing an aliphatic ring may also be used as the thermoplastic resin. For example, a thermoplastic resin that has at least any one of the following structural units may be used: a structural unit represented by Formula (23) below; and, as a monomer, an isosorbide-derived structural unit, a pentacyclopentadecane dimethanol (PCPMD)-derived structural unit, a cyclohexanedimethanol-derived structural unit, or a spiroglycol-derived structural unit.

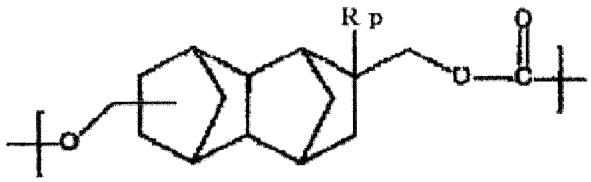

(23)

(In General formula (23), $R_p$ represents a hydrogen atom or an alkyl group with 1-4 carbon atoms.)

A copolymer or a blend of either a cyclo-olefin-based resin or a resin having a structural unit containing an aliphatic ring described above and a structural unit represented by General formula (24) below may also be used as a thermoplastic resin. Specific examples of such a thermoplastic resin include a copolymer or a blend having the structural unit represented by General formula (23) above and the structural unit represented by General formula (24) below.

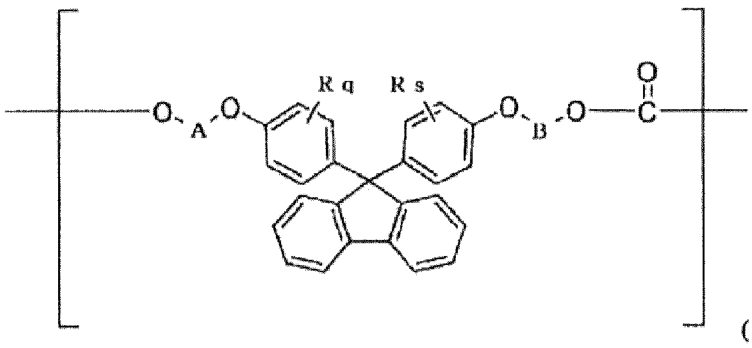

(24)

(In General formula (24), $R_q$ and $R_s$ each independently represent a hydrogen atom, an alkyl group with 1-20 carbon atoms, an alkoxyl group with 1-20 carbon atoms, a cycloalkyl group with 5-20 carbon atoms, a cycloalkoxyl group with 5-20 carbon atoms, an aryl group with 6-20 carbon atoms, or an aryloxy group with 6-20 carbon atoms, and A and B each independently represent an alkylene group with 1-4 carbon atoms.)

The thermoplastic resin may contain an acrylic resin. Examples of the acrylic resin include, but are not particularly limited to, a homopolymer of various (meth)acrylic esters typified by polymethyl methacrylate (PMMA) and methyl methacrylate (MMA), a copolymer of PMMA or MMA with one or more other monomers, and also a mixture of multiple kinds of these resins.

The weight-average molecular weight (Mw) of the thermoplastic resin in terms of polystyrene is preferably 10,000-300,000, more preferably 15,000-100,000, and still more preferably 20,000-50,000.

In addition, the viscosity-average molecular weight (Mv) of the thermoplastic resin is preferably 5,000-200,000,

more preferably 7,000-70,000, and still more preferably 10,000-30,000.

The thermoplastic resin such as a polycarbonate resin, a polyester resin, a polyester carbonate resin, a cycloolefin-based resin, or an acrylic resin in the thermoplastic resin composition is produced by a known method. Examples of a method for producing a thermoplastic resin include interfacial polymerization, melt transesterification, and condensation polymerization. Furthermore, examples of a method for producing a polycarbonate resin include ring-opening polymerization using a cyclic carbonate compound, pyridine method, and solid phase transesterification of a prepolymer.

The polycarbonate resin, polyester resin, or polyester carbonate resin produced by interfacial polymerization method using a chain terminator contains a terminal structure derived from the chain terminator. For example, the thermoplastic resin may have a terminal group such as a para-tert-butylphenyl group, a para-tert-octylphenyl group, or a p-benzoic acid hexadecane group, corresponding to the phenolic compound such as para-tert-butylphenol, para-tert-octylphenol, or para-hydroxybenzoic acid hexadecane, respectively, which is used as a chain terminator in the interfacial polymerization method. These terminal structures can improve the flowability of the thermoplastic resin such as a polycarbonate resin.

[2. Properties of thermoplastic resin composition]

A thermoplastic resin composition of the present invention containing the compounding agent can maintain transmissivity (%) at a higher level than thermoplastic resin compositions without the compounding agent.

For example, the value of transmissivity (%) of the thermoplastic resin composition at a wavelength of 370-400 nm according to JIS K7105 is higher than that of a reference resin composition having the same composition but without the compounding agent, by 2.0 (%) or more. Specifically, when the values of transmissivity (%) of a reference resin composition without the compounding agent at wavelengths of 370-400 nm according to JIS K7105 were compared to the values of transmissivity (%) of the thermoplastic resin composition of the present invention at the same wavelengths, i.e., 370-400 nm, according to JIS K7105, the values of transmissivity were higher for the thermoplastic resin composition and a difference of 2.0 (%) or more was realized between these values. The value of transmissivity of the thermoplastic resin composition is higher than the value of transmissivity of the reference resin composition preferably by 3.0 (%) or more, and more preferably by 4.0 (%) or more.

For example, the value of transmissivity (%) of the thermoplastic resin composition at a wavelength of 370-400 nm according to JIS K7105 is 1.1 times or more higher than that of a reference resin composition having the same composition but without the compounding agent. Specifically, when the values of transmissivity (%) of a reference resin composition without the compounding agent at wavelengths of 370-400 nm according to JIS K7105 were compared with the values of transmissivity (%) of the thermoplastic resin composition of the present invention at the same wavelengths, i.e., 370-400 nm, according to JIS K7105, the values of transmissivity of the thermoplastic resin composition were higher than those of the reference resin composition and the difference thereof was 1.1 times or more. The value of transmissivity of the thermoplastic resin composition is preferably 1.3 times or more and more preferably 1.5 times or more than the value of transmissivity of the reference resin composition.

The addition of additives, for example, a mold release agent, an antioxidant, and the like, to the thermoplastic resin composition of the present invention tends to deteriorate the transmissivity value of the resulting thermoplastic resin composition. However, with a thermoplastic resin composition to which the above-mentioned compounding agent has been added, a decrease in the transmissivity value can be prevented and suppressed.

Moreover, a thermoplastic resin composition in one aspect of the present invention has a lower haze value according to JIS K-7361 and JIS K-7136 than a reference thermoplastic resin composition which has the same composition as said thermoplastic resin composition but without the compounding agent, where the difference between these haze values is 0.03 or more. Thus, the above-mentioned compounding agent is also found to improve the transparency of the thermoplastic resin composition.

The difference between the haze value of the thermoplastic resin composition and that of the reference resin composition is preferably 0.05 or more, more preferably 0.07 or more, still more preferably 0.10 or more, and particularly preferably 0.12 or more. In addition, the thermoplastic resin composition in one aspect of the present invention has a haze value that is lower than the haze value of the reference resin composition by 0.15 or more. The thermoplastic resin composition in one aspect of the present invention preferably has a haze value that is lower than the haze value of the reference resin composition by 0.18 or more or 0.20 or more.

Moreover, a thermoplastic resin composition in one aspect of the present invention has a lower YI value than that of the reference thermoplastic resin composition having the same composition as said thermoplastic resin composition but without the compounding agent. Specifically, the YI value of a thermoplastic resin composition containing the above-described compounding agent, for example, according to JIS K 7105, can be 0.20 or more lower than that of a thermoplastic resin composition which has the same composition but without the compounding agent. The difference between these YI values can be, for example, 0.50 or more, 0.80 or more, 0.90 or more, 1.0 or more, or even 1.1 or more.

As described above, the thermoplastic resin composition of the present invention, which can maintain high

transmissivity and have improved color hue as indicated by a low YI value, is suitable for applications such as an optical material. The thermoplastic resin composition of the present invention can suitably be used especially as an optical material.

Furthermore, the thermoplastic resin composition of the present invention has high heat resistance and transparency, and is expected to have a reduced amount of volatile components. Among the thermoplastic resin compositions of the present invention, the effect of reducing the amount of a volatile component at high temperatures has been confirmed especially in the polyester resin composition as will be described in detail below and thus the odor generated during heating can be suppressed. For this reason, the thermoplastic resin compositions of the present invention, especially the polyester resin compositions, are useful, for example, as a plastic material for food containers and packaging.

For example, a thermoplastic resin composition in one aspect of the present invention is as effective as or more effective than a reference thermoplastic resin composition which has the same composition as said thermoplastic resin composition but without the compounding agent in suppressing a volatile component which will be described in detail below. In other words, the thermoplastic resin composition can have a suppressed amount of volatile component, which is generated under certain conditions, e.g., heating at 250°C for 5 minutes.

Specific examples of the volatile component include formaldehyde, acetaldehyde, acetone, 2,3-butanedione, acetic acid, and formic acid.

[3. Method for producing thermoplastic resin composition]

A method for producing a thermoplastic resin composition for use as an optical material according to the present invention includes a step of adding the above-described compounding agent to a thermoplastic resin. By the step of adding the compounding agent to a thermoplastic resin, the transmissivity of the thermoplastic resin, especially at a low wavelength, can be maintained better than that of a thermoplastic resin to which the compounding agent is not added.

[4. Method for improving transmissivity of thermoplastic resin composition]

A method for improving the transmissivity of a thermoplastic resin composition for use as an optical material according to the present invention includes a step of adding the above-described compounding agent to a thermoplastic resin. By the step of adding the compounding agent to a thermoplastic resin, the transmissivity of the thermoplastic resin, especially at a low wavelength, can be improved. In particular, the transmissivity value can be improved in a thermoplastic resin composition to which the compounding agent is further added compared to a thermoplastic resin to which additives other than the compounding agent are added.

[5. Method for lowering haze of thermoplastic resin composition]

A method for lowering the haze of a thermoplastic resin composition of the present invention comprises a step of adding the above-described compounding agent to a thermoplastic resin. By the step of adding the compounding agent to a thermoplastic resin, the haze value of the thermoplastic resin, especially the haze value according to JIS K-7361 and JIS K-7136, can be lowered. Specifically, the haze value of the thermoplastic resin composition is lower than that of a reference resin composition, which has the same composition as the thermoplastic resin composition but without the compounding agent. The difference between these haze values is, for example, 0.03 or more, 0.05 or more, 0.07 or more, 0.10 or more, 0.12 or more, 0.15 or more, 0.18 or more, or 0.20 or more as mentioned above, and may be 0.30 or more or 0.40 or more.

[6. Molded article]

The thermoplastic resin composition of the present invention can be subjected to extrusion molding, blow molding, injection molding, and the like.

Examples of the resulting molded article include extruded articles, hollow molded articles, and injection molded articles such as precision parts and thin parts.

The thermoplastic resin composition of the present invention can maintain a good transmissivity value. For this reason, the thermoplastic resin composition of the present invention is particularly suitable as an optical material. Examples of the molded article that can be manufactured using such a thermoplastic resin composition include an optical lens, an optical film, a liquid crystal display, an organic EL display, a transparent conductive substrate used in a solar cell, etc., an optical disc, a liquid crystal panel, an optical card, a sheet, a film such as a retardation film, optical fiber, a connector, a deposited plastic reflective mirror, a display, and a touch panel. These molded articles for optical use have high transmissivity even when they contain additives added for various applications.

Specific examples of the molded article that uses the thermoplastic resin of the present invention as an optical material or for an application in related fields include optical media such as a compact disc, a digital video disc, a mini disc, and a magneto-optical disc, optical communication media such as optical fiber, an optical component such as a headlamp lens for cars and a lens for cameras, a siren light cover, a lighting lamp cover, an alternative to window glass for vehicles (e.g., trains and automobiles), an alternative to window glass for households, daylighting parts such as a sunroof and a greenhouse roof, a lens or a case for goggles, sunglasses, and eyeglasses, a housing for office automation equipment such as a copier and a facsimile, a housing for a household appliance such as a television and a microwave oven, and an electronic component such as a connector and an IC tray. Other examples include, but are not limited to, protective equipment such as a helmet, a protector, and a protective mask, household goods such as a baby bottle, a dish, and a tray, a medical supply such as a case for dialysis and a denture, a packaging material, and miscellaneous goods such as a writing tool and stationery.

Examples of particularly favorable molded articles that are obtained using the thermoplastic resin composition of the present invention include the following, which require high heat resistance and high transparency. Specifically, a headlamp lens, a meter panel, a sunroof, and other automotive component, as well as an alternative to a glass window and outer panel parts; various films for a liquid crystal display or the like, a light guide plate, an optical disc substrate, and a housing for a smartphone or other electronic device; and a construction material such as a transparent sheet.

Even for molded articles that do not strictly require the excellent transparency of the thermoplastic resin composition of the present invention, the high transparency of the resin composition as the raw material is found to be beneficial in making it easier to control the degree of coloring using a coloring agent such as a pigment or dye.

In addition, if necessary, the surface of the molded optical article may also be provided with a coating layer, such as an anti-reflective layer or hard coat layer. The anti-reflective layer may be either a single- or multi-layer, and it may be either organic or inorganic, preferably inorganic. Specifically, examples include oxides and fluorides such as silicon oxide, aluminum oxide, zirconium oxide, titanium oxide, cerium oxide, magnesium oxide, and magnesium fluoride.

[6-1. Optical Lens]

An optical lens manufactured using the thermoplastic resin composition of the present invention will have excellent transmissivity and will also have a high refractive index, low Abbe number, and high moist heat resistance, and therefore it is extremely useful for use in telescopes, binoculars, television projectors, and other fields where expensive high-refractive-index glass lenses are conventionally used. If necessary, the optical lens is suitably used in the form of an aspheric lens. Since spherical aberration can be reduced to virtually zero with a single aspherical lens, there is no need to remove spherical aberration by combining multiple spherical lenses, which enables weight reduction and production cost reduction. Therefore, an aspherical lens is especially useful as a camera lens among other optical lenses.

Optical lenses can be molded by any method, such as injection molding, compression molding, or injection-compression molding. According to the present invention, an aspheric lens with a high refractive index and low birefringence, which is technically difficult to process from a glass lens, can be obtained more easily.

To avoid contamination of the optical lens with foreign matter as much as possible, the molding environment obviously needs to be a low dust environment, preferably class 6 or better, and more preferably class 5 or better.

An optical lens produced using the thermoplastic resin composition of the present invention can be obtained by injection molding the above-described polycarbonate copolymer of the present invention into a lens shape using an injection molding machine or injection compression molding machine. While the conditions for injection molding are not limited, the molding temperature is preferably 180-280°C. In addition, the injection pressure is preferably 50-1,700 kg/cm$^2$.

To avoid contamination of the optical lens with foreign matter as much as possible, the molding environment obviously needs to be a low dust environment, preferably class 1,000 or better, and more preferably class 100 or better.

An optical lens containing the thermoplastic resin composition of the present invention is suitably implemented in the form of an aspherical lens as necessary. Since spherical aberration can be reduced to virtually zero with a single aspherical lens, there is no need to remove spherical aberration by combining multiple spherical lenses, which enables weight reduction and production cost reduction. Therefore, the aspherical lens is especially useful as a camera lens among other optical lenses. The astigmatism of the aspheric lens is preferably 0-15 m$\lambda$, and more preferably 0-10 m$\lambda$.

The thickness of the optical lens manufactured using the thermoplastic resin composition of the present invention can be set within a wide range depending on the application and is not particularly limited, but it is preferably 0.01-30 mm, and more preferably 0.1-15 mm. If necessary, the surface of the optical lens of the present invention may also be provided with a coating layer, such as an anti-reflective layer or hard coat layer. The anti-reflective layer may be either a single- or multi-layer, and it may be either organic or inorganic, preferably inorganic. Specifically, examples include oxides or fluorides such as silicon oxide, aluminum oxide, zirconium oxide, titanium oxide, cerium oxide, magnesium oxide, and magnesium fluoride. Among these, silicon oxide and zirconium oxide are preferable, and a combination of silicon oxide and zirconium oxide is more preferable. As to the anti-reflective layer, the combination of single/multi-layers, the composition and thickness thereof, and else are not particularly limited, but it preferably has a two- or three-layer

structure, particularly preferably a three-layer structure. The anti-reflective layer as a whole is preferably formed to have a thickness of 0.00017-3.3% of the thickness of the optical lens, specifically 0.05-3 μm, and particularly preferably 1-2 μm.

[6-2. Optical film]

An optical film produced using the thermoplastic resin composition of the present invention has excellent transparency and thermal stability, it is suitable for use in a LCD substrate film, an optical memory card, and other applications.
To avoid contamination of the optical film with foreign matter as much as possible, the molding environment obviously needs to be a low dust environment, preferably class 6 or better, and more preferably class 5 or better.

EXAMPLES

Hereinafter, the invention will be described more specifically by means of examples. The present invention, however, is not limited to the following examples, and may be modified and carried out as desired within the scope not departing from the gist of the present invention.

[Evaluation methods 1]

Methods of evaluation employed in the examples and comparative examples using polycarbonate resins, which will be described in detail below, were as follows.

(1-1) Transmissivity [%]

Pellets of a thermoplastic resin composition obtained by the following method were dried in a hot-air circulating dryer at 120°C for 5 hours, and then molded into a flat test plate piece that was 40 mm wide × 40 mm long × 3 mm thick using an injection molding machine (ROBOSHOT S-2000i30A, manufactured by Fanuc Corporation) under the following conditions: resin temperature of 260°C, mold temperature of 130°C, and molding cycle of 30 seconds. The transmissivities (%) of a 3 mm-thick part of the flat test plate piece were measured at wavelengths of 370 nm, 380 nm, and 400 nm using a spectrophotometer ("U-4100", manufactured by Hitachi High-Technologies Corporation) according to JIS K7105.

(1-2-1) Mass-average molecular weight (Mw)

The mass-average molecular weights of the resins and resin composition were measured by gel permeation chromatography (GPC) and determined in terms of polystyrene. The apparatus, columns, and measurement conditions used were as follows.

·GPC apparatus: HLC-8420GPC, manufactured by Tosoh Corporation
·Columns: TSKgel SuperHM-M, manufactured by Tosoh Corporation, x 3 columns
TSKgel guardcolumn SuperH-H, manufactured by Tosoh Corporation, x 1 column
TSKgel SuperH-RC, manufactured by Tosoh Corporation, x 1 column
·Detector: RI detector
·Standard polystyrenes: Standard polystyrene kit PStQuick C, manufactured by Tosoh Corporation
·Sample solution: 0.2 mass% tetrahydrofuran solution
·Eluent: tetrahydrofuran
·Flow rate of eluent: 0.6 mL/min
·Column temperature: 40°C

(1-2-2) Viscosity-average molecular weight (Mv)

The viscosity-average molecular weight (Mv) of the thermoplastic resin was calculated by Schnell's viscosity formula, i.e., $\eta = 1.23 \times 10^{-4}\ Mv^{0.83}$.
The value of intrinsic viscosity [η] (unit dL/g) can be obtained by the following equation.

$$\eta = \lim_{c \to 0} \eta_{sp} / c$$

The value of specific viscosity [$\eta_{sp}$] in the above formula is a value obtained by dissolving the resin in methylene chloride at various concentrations [C] (g/dL) to prepare samples, and measuring the specific viscosities of the samples with a Ubbelohde viscometer at 25°C. From the thus-obtained specific viscosities [$\eta_{sp}$] and the concentrations [C], the intrinsic viscosity [$\eta$] (unit: dL/g) is calculated by the above formula.

<Synthesis of thermoplastic resin: PC1>

As raw materials, 20.86 kg (47.56 mol) of 9,9-bis[4-(2-hydroxyethoxy)-phenyl]fluorene (BPEF), 10.5 kg (49.02 mol) of diphenyl carbonate (DPC), and 16 milliliters of $2.5 \times 10^{-2}$ mol/liter aqueous sodium bicarbonate solution ($4.0 \times 10^{-4}$ mol, i.e., $8.4 \times 10^{-6}$ mol per 1 mol of the total dihydroxy compound) were placed in a 50 L reactor equipped with a stirrer and a distillation unit and heated to 180°C in a nitrogen atmosphere at 760 mmHg. After 30 minutes of heating, complete dissolution of the raw materials was confirmed, and then the mixture was stirred for 120 minutes under the same conditions. The pressure was then reduced to 200 mmHg while the temperature was simultaneously increased to 200°C at a rate of 60°C/hr. During this, distillation of the phenol as the byproduct was confirmed to have started. Thereafter, the temperature was maintained at 200°C for 20 minutes to allow the reaction to take place. The temperature was further increased to 230°C at a rate of 75°C/hr. Ten minutes after the end of the temperature increase, the pressure reduction was reduced to 1 mmHg or less by spending 2 hours while maintaining this temperature. The temperature was then increased to 245°C at a rate of 60°C/hr and stirring was performed for another 40 minutes. At the end of the reaction, nitrogen was introduced into the reactor to recover normal pressure. The generated resin was taken out from the reactor while pelletizing to obtain a thermoplastic resin, i.e., polycarbonate resin (PC1).

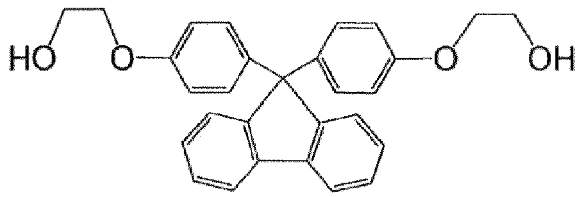

(BPEF)

<Synthesis of thermoplastic resin: PC2>

A thermoplastic resin, i.e., polycarbonate resin (PC2), was obtained in the same manner as Synthesis example 1 except that the raw materials were changed to 14.978 kg (40.000 mol) of 2,2'-bis(2-hydroxyethoxy)-1,1'-binaphthalene (BNE), 24.239 kg (45.000 mol) of 9,9-bis[6-(2-hydroxyethoxy)naphthalene-2-yl]fluorene (BNEF), 7.899 kg (15.000 mol) of DPBHBNA, 22.236 kg (103.800 mol) of DPC, and $5.09 \times 10^{-2}$ g ($6.06 \times 10^{-4}$ mol) of sodium bicarbonate.

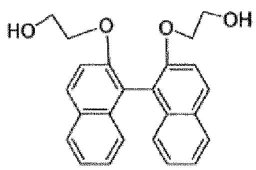

(BNE)

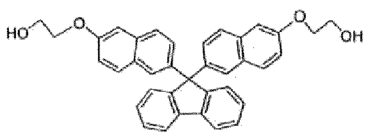

(BNEF)

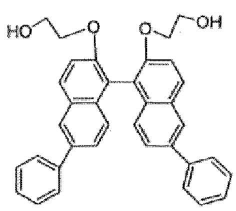

(DPBHBNA)

<Examples 1-3, etc.>

PC1 obtained in Synthesis example 1, additives (mold release agent, antioxidant), and a compounding agent were mixed in the mass ratios shown in Table 1 below in a dry process using a tumbler, melt-kneaded using a twin-screw extruder (IPT-type 35 mm co-rotating twin-screw extruder, LID = 38, manufactured by IPEC) at a cylinder temperature of 250°C, vent pressure of 25 Torr, and discharge rate of 20 kg/hour, and extruded as strands to obtain pelletized thermoplastic resins, i.e., polycarbonate resin compositions. The transmissivities of the obtained resin compositions

were measured and the results are shown in Table 1.

[Table 1]

| | | | Example 1 | Example 2 | Example 3 | Comparative example 1 | Reference example |
|---|---|---|---|---|---|---|---|
| Resin | | | PC1 | PC1 | PC1 | PC1 | PC1 |
| Mold release agent [ppm] | | S-100A | 1,000 | 1,000 | 0 | 1,000 | 1,000 |
| Antioxidant | Phosphite-based [ppm] | PEP-36 | 200 | 200 | 200 | 200 | 0 |
| | Phenolic [ppm] | AO-60 | 1,000 | 0 | 1,000 | 1,000 | 0 |
| Compounding agent [ppm] | | | 200 | 200 | 200 | 0 | 200 |
| Transmissivity [%] | 400 nm | | 84.35 | 84.07 | 84.38 | 82.32 | 78.79 |
| | 380 nm | | 81.60 | 81.02 | 81.62 | 79.50 | 69.90 |
| | 370 nm | | 79.10 | 78.42 | 79.11 | 76.92 | 64.56 |

Antioxidant AO-60:
Pentaerythritol-tetrakis[3-(3,5-di-tert-butyl-4-hydroxyphenyl)propionate] (AO-60, manufactured by ADEKA Corporation)
Antioxidant PEP-36:
3,9-Bis(2,6-di-tert-butyl-4-methylphenoxy)-2,4,8,10-tetraoxa-3,9-diphosphaspiro [5.5]undecane (PEP-36, manufactured by ADEKA Corporation)
Mold release agent S-100A:

Glycerol monostearate (S-100A, manufactured by Riken Vitamin Co., Ltd.)
Compounding agent: A commercially available product manufactured by Tokyo Chemical Industry Co., Ltd., a mixture represented by the following formula (90:10 mixture of 3,4-dimethyl and 2,4-dimethyl)

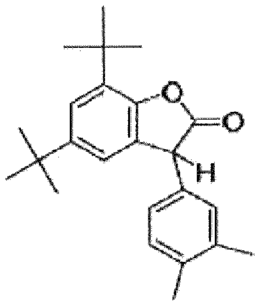
90%
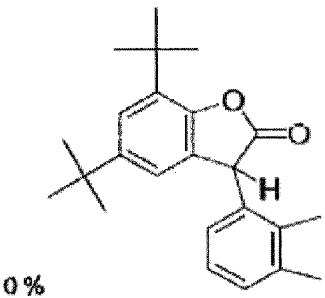
10%

<Examples 4-7, etc.>

Pelletized thermoplastic resins, i.e., polycarbonate resin compositions, were obtained in the same manner as Example 1 except that PC2 obtained in Synthesis example 2, additives (mold release agent, antioxidant), the compounding agent, and a catalyst deactivator were used in the mass ratios shown in Table 2 below. The transmissivities of the obtained resin compositions were measured and the results are shown in Table 2. Abbreviations in Table 2, except for MGA, which stands for tetrabutylphosphonium dodecylbenzene sulfonate, are as indicated in the margin of Table 1.

[Table 2]

| | Example 4 | Example 5 | Example 6 | Example 7 | Comparative example 4 |
|---|---|---|---|---|---|
| Resin | PC2 | PC2 | PC2 | PC2 | PC2 |

(continued)

| | | | Example 4 | Example 5 | Example 6 | Example 7 | Comparative example 4 |
|---|---|---|---|---|---|---|---|
| Mold release agent [ppm] | | S-100A | 1,500 | 1,500 | 1,500 | 0 | 1,500 |
| Antioxidant | Phosphite-based [ppm] | PEP-36 | 300 | 300 | 300 | 300 | 300 |
| | Phenolic [ppm] | AO-60 | 1,000 | 1,000 | 1,000 | 1,000 | 1,000 |
| Compounding agent [ppm] | | | 200 | 1,500 | 200 | 200 | 0 |
| Catalyst deactivator/Water [ppm] | MGA/Wat er | | 15/1000 | 15/1000 | 0 | 15/1000 | 15/1000 |
| Transmissivity [%] | 400 nm | | 25.91 | 25.08 | 21.03 | 25.92 | 19.28 |
| | 380 nm | | 0.98 | 0.91 | 074 | 0.98 | 0.47 |
| | 370 nm | | 0.32 | 0.31 | 0.29 | 0.32 | 0.29 |

(1-3) Total transmissivity and haze

Resin composition samples of the following examples and comparative examples, which were molded to have a thickness of 3 mm, were subjected to measurements according to JIS K-7361 and JIS K-7136.

Measurement equipment: Spectral haze meter SH7000, manufactured by Nippon Denshoku Industries Co., Ltd.

(1-4) YI

The samples molded to have a thickness of 3 mm were subj ected to a measurement according to JIS K-7105 using a spectral haze meter.

Measurement equipment: Spectral haze meter SH7000, manufactured by Nippon Denshoku Industries Co., Ltd.

<Examples 8-10, etc.>

PC3 (cyclic olefin copolymer "APEL" (registered trademark), brand name: APL5014CL, manufactured by Mitsui Chemicals Inc.), additives (mold release agent, antioxidant), and the compounding agent were mixed in the mass ratios shown in Table 3 below in a dry process using a tumbler, melt-kneaded using a twin-screw extruder (trade name "TEM18ss", manufactured by Toshiba Machine Co., Ltd.) at a cylinder temperature of 260°C, and extruded as strands to obtain pelletized thermoplastic resins, i.e., polycarbonate resin compositions.

The physical properties of the obtained resin compositions were measured and the results are shown in Table 3.

The molecular structure of the cyclic olefin copolymer in PC3 above is represented by Formula (22) above.

[Table 3]

| | | | Example 8 | Example 9 | Example 10 | Comparative example 5 | Comparative example 6 |
|---|---|---|---|---|---|---|---|
| Resin | | | PC3 | PC3 | PC3 | PC3 | PC3 |
| Antioxidant | Phosphite-based [ppm] | PEP-36 | 500 | 500 | 500 | 500 | 0 |
| | Phenolic [ppm] | AO-60 | 0 | 0 | 2,500 | 5,000 | 0 |
| Compounding agent [ppm] | | | 3,000 | 5,000 | 2,500 | 0 | 0 |
| Physical properties | YI | | 3.14 | 3.46 | 3.41 | 3.34 | 3.37 |
| | Haze | | 0.43 | 0.35 | 0.43 | 0.55 | 0.51 |
| | Total transmissivity [%] | | 90.09 | 89.82 | 90.16 | 90.55 | 90.69 |
| | Spectral transmissivity [%] | 400 nm | 79.75 | 77.95 | 79.66 | 82.16 | 82.11 |
| | | 380 nm | 70.12 | 66.71 | 70.55 | 75.96 | 75.81 |

As to Examples 8-10, etc., in which a cyclic olefin copolymer having the above-described molecular structure was used, although the effect of lowering the transmissivity in the low wavelength range was not as pronounced as in other examples, the haze property was improved. In addition, the YI values were also kept low and excellent hue was confirmed for the polycarbonate resin compositions of Examples 8-10.

(1-5) Powder X-ray diffraction

For powder X-ray diffraction of the crystal of the above-described compounding agent that was used in Example 1 and else (90:10 mixture of 3,4-dimethyl and 2,4-dimethyl, i.e., two of $R_1$-$R_5$ were methyl groups while the other two were hydrogen, and two of $R_6$-$R_9$ were tertiary butyl groups while the other two were hydrogen in General formula (1), respectively), the measurement conditions were as follows.

A sample holding part of a glass slide was filled with the crystal to be measured in an appropriate quantity, and measurement was performed using a powder X-ray diffractometer (MiniFlex 600, manufactured by Rigaku Corporation) under the following conditions.

X-ray: CuK$\alpha$ (40 kV, 15 mA)
K$\beta$ filter: Ni filter 0.015 mm × 1 piece
Scan speed: 10°/min
Step width: 0.02°
Scan axis: 2$\theta$/$\theta$
Scan range: 5°-90°
Incident slit (DS): 1.25°
Height limiting slit (IHS): 10.0 mm
Receiving slit 1 (SS): 8.0 mm
Receiving slit 2 (RS): 13.0 mm

Powder X-ray diffraction measurements were performed for three different lots of the above compounding agent (Samples 1-3). Sample 1 was the one actually employed in Example 1, and Samples 2 and 3 were the same compounds as Sample 1 but were separately manufactured.

As the results of the measurements, the presence of peaks at 6.7°, 10.4°, 11.1°, 12.7°, 16.1 ± 0.2°, 17.3 ± 0.2°, 20.8°, and 23.6° in addition to the main peaks at 13.2°, 15.2°, and 20.8° were confirmed (see Table 4 below and Figures 1-3).

As can be appreciated from Table 4 below, the peak values were generally the same among the separate samples, but measurement errors of about ±0.2° or ±0.1° may occur.

[Table 4]

| Table 4-A Sample 1 | | | Table 4-B Sample 2 | | | Table 4-C Sample 3 | |
|---|---|---|---|---|---|---|---|
| Diffraction angle (2$\theta$, °) | Relative intensity | | Diffraction angle (2$\theta$, °) | Relative intensity | | Diffraction angle (2$\theta$, °) | Relative intensity |
| 6.62 | 6,690 | | 6.74 | 17,000 | | 6.66 | 4,870 |
| 10.4 | 20,900 | | 10.5 | 22,500 | | 10.4 | 22,600 |
| 11.1 | 12,700 | | 11.1 | 12,900 | | 11.1 | 11,900 |
| 12.7 | 8,300 | | 12.7 | 8,450 | | 12.7 | 10,600 |
| 13.2 | 45,200 | | 13.3 | 52,600 | | 13.2 | 42,100 |
| 15.2 | 36,700 | | 15.3 | 41,200 | | 15.2 | 36,200 |
| 16.1 | 24,900 | | 16.1 | 26,100 | | 16.1 | 28,800 |
| 17.3 | 25,400 | | 17.4 | 25,000 | | 17.3 | 27,800 |
| 20.8 | 44,800 | | 20.9 | 53,600 | | 20.8 | 45,700 |
| 23.6 | 10,900 | | 23.7 | 10,400 | | 23.6 | 12,300 |

[Evaluation methods 2]

Methods for evaluating polyester resins and polyester resin compositions of the examples, which will be described in detail below, were as follows.

(2-1) Percentages of diol unit having cyclic acetal skeleton and alicyclic diol unit

The percentages of a unit derived from diol having cyclic acetal skeleton and a unit derived from alicyclic diol in the polyester resin were determined by $^1$H-NMR measurement. Measurement was performed using a measurement apparatus Ascend TM500 manufactured by Bruker BioSpin K.K. Deuterated chloroform was used as the solvent.

(2-2) Glass transition temperature

The glass transition temperature (Tg) of the polyester resin was measured using a differential scanning calorimeter (model: DSC/TA-50WS) manufactured by Shimadzu Corporation, with approximately 10 mg of a sample placed in an unsealed aluminum container and at a temperature increase rate of 20°C/min in a nitrogen gas flow (30 ml/min). The glass transition temperature was defined as the temperature at which the DSC curve changed by half of the difference between the baselines before and after the transition.

(2-3) YI value of pellets

The YI value was measured according to JIS K-7105 using "ZE2000" manufactured by Nippon Denshoku Industries Co., Ltd.

(2-4) Amounts of volatile components upon melting pellets

The relative value of the peak area of a low molecular weight compound (volatile component) obtained by GC-MS (headspace) converted per unit weight of the sample, i.e., the relative value of the peak area of a volatile component in each example when the peak area value of the volatile component in Comparative example 5 or 8 (described below) corresponding to said example was 100%, was used as the amount of the volatile component (%).

Specifically, the measurements was carried out as follows.

0.3 g of dried pellets were placed in a HS vial and sealed with a septum under air. After heating to 250°C for 5 minutes in a block heater, headspace GC-MS analysis was immediately started. In the analysis, a mass chromatogram was extracted with ions that are characteristic of each compound, and the peak area value per unit weight was obtained for each sample. The apparatus used and the measurement conditions were as follows.

[HS] Agilent G1888
Heating temperature and time: 250°C, 5 min. (external thermostat)
+230°C, 1 min.
Loop temperature: 240°C
TR LINE temperature: 250°C
Vial equilibration: 1 min., vial pressurization: 0.5 min. (15 psi)
Loop fill: 0.2 min., loop equilibration: 0.2 min., injection: 0.1 min.
GC Cycle: Analysis 35 min. + Equilibration 10 min.
Carrier pressure: 16.5 psi

[GC] Agilent 8890
Column: DB-WAX ($\Phi$0.25 mm $\times$ 60 $\times$ t0.5 $\mu$m)
Oven temperature: 40°C for 5 min., then heating at 10°C/min until 240°C (hold for 10 min.)
Column flow: He 1.0 ml/min
Split ratio: 1/10
Injection temperature: 240°C
MSD transfer line: 240°C

[MS] Agilent 5977B MSD
Gain factor: 1
Scan range: m/z = 29-700

<Examples 11-14, etc.>

Hereinafter, Examples 11-14, etc. using polyester resins will be described.

<Production example>

[Synthesis of polyester resins (PEs-1 and PEs-2)]

The raw monomers listed in Table 5 below were fed into a 30 L polyester manufacturing apparatus equipped with a packed column-type distillation column, partial condenser, total condenser, cold trap, stirrer, heating device, and nitrogen inlet tube, and 0.005 mol% tetra-n-butoxytitanium and 0.02 mol% potassium acetate were added to the dicarboxylic acid component. The temperature was raised to 225°C in a nitrogen atmosphere to allow transesterification reaction. Once the reaction conversion rate of the dicarboxylic acid component reached 90% or higher, 0.025 mol% germanium dioxide and 0.05 mol% triethyl phosphate were added to the dicarboxylic acid component, and the temperature was increased and the pressure was decreased gradually, finally resulting in polycondensation at 280°C and 0.1 kPa or lower. The reaction was terminated once the melt viscosity reached an appropriate level, thereby synthesizing PEs-1 or PEs-2 polyester resin.

[Table 5]

| | Production example 1 (PEs-1) | Production example 2 (PEs-2) |
|---|---|---|
| Monomer feed<br>Dicarboxylic acid component (mol)<br>DMT<br>NDCM | <br><br>0<br>31.2 | <br><br>49.8<br>0 |
| Diol component (mol)<br>EG<br>SPG<br>CHDM | <br>35.9<br>13.0<br>20.3 | <br>89.6<br>23.3<br>0 |
| Results from evaluation of polyester resin | | |
| Percentage of unit derived from diol having cyclic acetal skeleton (mol%) | 38.9 | 44.3 |
| Percentage of unit derived from alicyclic diol (mol%) | 53.3 | 0 |
| Glass transition temperature (°C) | 135 | 110 |

Abbreviations used in Table 5 are as follows.

DMT: Dimethyl terephthalate
NDCM: Dimethyl 2,6-naphthalenedicarboxylate
EG: Ethylene glycol
SPG: 3,9-Bis(1,1-dimethyl-2-hydroxyethyl)-2,4,8,10-tetraoxaspiro[5.5]undecane
CHDM: 1,4-Cyclohexanedimethanol

(Preparation of kneaded pellets)

Using a twin-screw extruder (manufactured by Technobell, model: KZW15TW-30MG-NH (-700), screw diameter: 15 mmcp, L/D: 30), either polyester resin PEs-1 or PEs-2 synthesized in the above production example and an antioxidant were dry-blended at a predetermined ratio and the mixture was fed from the hopper. A strand was extruded under the following conditions: a cylinder temperature of 210-280°C, a die temperature of 280°C, a screw speed of 60 rpm, and a discharge rate of 1.4 kg/h. After cooling in the air, the strand was pelletized to obtain raw polyester resin pellets and pellets kneaded with additives. The kinds and amounts of the additives are shown in Tables 6 and 7.

The additives used were as follows.

Antioxidant Irganox 1330:

3,3',3",5,5',5"-Hexa-tert-butyl-α,α',α"-(mesitylene-2,4,6-triyl)tri-p-cresol (Irganox 1330, manufactured by BASF Japan Ltd.)

Antioxidant PEP-36:

3,9-Bis(2,6-di-tert-butyl-4-methylphenoxy)-2,4,8,10-tetraoxa-3,9-diphosphaspiro [5,5]undecane (PEP-36, manufactured by ADEKA Corporation)

Compounding agent: A commercially available product manufactured by Tokyo Chemical Industry Co., Ltd., a mixture represented by the following formula (90:10 mixture of 3,4-dimethyl and 2,4-dimethyl).

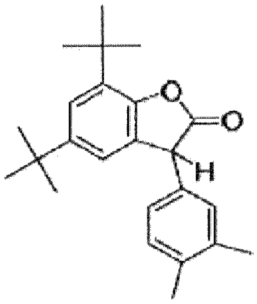
90%

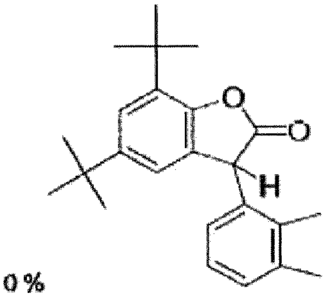
10%

[Table 6]

| Resin | | | Example 11 | Example 12 | Comparative example 7 | Comparative example 8 | Comparative example 9 |
|---|---|---|---|---|---|---|---|
| | | | PEs-1 | PEs-1 | PEs-1 | PEs-1 | PEs-1 |
| Antioxidant | Phosphite-based [ppm] | PEP-36 | 5,000 | 5,000 | 0 | 0 | 5,000 |
| | Phenolic [ppm] | Irganox 1330 | 0 | 3,000 | 3,000 | 0 | 3,000 |
| Compounding agent [ppm] | | | 500 | 500 | 0 | 0 | 0 |
| Physical property | YI | | 13.6 | 16.2 | 18.5 | 17.8 | 16.5 |
| Amounts of volatile components (Peak area value converted per unit weight: Relative value (%) when Comparative Example 8 is 100%) | Formaldehyde | | 7 | 8 | 11 | 100 | 8 |
| | Acetaldehyde | | 50 | 62 | 60 | 100 | 60 |
| | Acetone | | 3 | 3 | 10 | 100 | 5 |
| | 2,3-Butanedione | | 7 | 7 | 73 | 100 | 12 |
| | Acetic acid | | 37 | 31 | 38 | 100 | 38 |
| | Formic acid | | 1 | 1 | 6 | 100 | 3 |

[Table 7]

| Resin | | | Example 13 | Example 14 | Comparative example 10 | Comparative example 11 |
|---|---|---|---|---|---|---|
| | | | PEs-2 | PEs-2 | PEs-2 | PEs-2 |
| Antioxidant | Phosphite-based [ppm] | PEP-36 | 5,000 | 5,000 | 0 | 0 |
| | Phenolic [ppm] | Irganox 1330 | 0 | 3,000 | 3,000 | 0 |
| Compounding agent [ppm] | | | 500 | 500 | 0 | 0 |
| Physical property | YI | | 10.2 | 9.2 | 20.6 | 16.2 |
| Amounts of volatile components (Peak area value converted per unit weight: Relative value (%) when Comparative Example 11 is 100%) | Formaldehyde | | 1 | 1 | 4 | 100 |
| | Acetaldehyde | | 19 | 21 | 10 | 100 |
| | Acetone | | 1 | 1 | 5 | 100 |
| | 2,3-Butanedione | | 7 | 7 | 64 | 100 |
| | Acetic acid | | 6 | 6 | 9 | 100 |
| | Formic acid | | 0 | 0 | 1 | 100 |

Compared to the polyester resin compositions according to Comparative examples 7-11 without the compounding agent, the effects of lowering YI and reducing volatile components in the kneaded pellets were observed in the polyester resin compositions according to Examples 11-14 to which the compounding agent has been added.

Furthermore, although the effect of reducing the amounts of volatile components in some examples were generally the same as those in the comparative examples (Example 12 and Comparative example 9), but a reduction effect owing to the addition of the compounding agent was still confirmed.

<Examples 15-18, etc.>

Hereinafter, Examples 15-18, etc. using bisphenol-based polycarbonate resins will be described.

Pelletized thermoplastic resins, i.e., polycarbonate resin compositions, were obtained in the same manner as Example 1 except that the following polycarbonate resins, additives (mold release agent, antioxidant), and compounding agent were used in the mass ratios shown in Tables 8-11 below. The physical properties obtained are shown in Tables 8-11.

Example 15

PCa: Trade name Iupizeta T-3840 (viscosity-average molecular weight Mv: 13,500) manufactured by Mitsubishi Gas Chemical Company, Inc., which was a bisphenol A-type aromatic polycarbonate having a p-t-octylphenyl group as a terminal structure

[Table 8]

| | | | Example 15-1 | Example 15-2 | Comparative example 12-1 | Comparative example 12-2 | Comparative example 12-3 |
|---|---|---|---|---|---|---|---|
| Resin | | | PCa | PCa | PCa | PCa | PCa |
| Mold release agent [ppm] | | S-100A | 1,000 | 1,000 | 1,000 | 1,000 | 1,000 |
| Antioxidant | Phosphite-based antioxidant [ppm] | PEP-36 | 500 | 500 | 500 | 500 | 0 |
| | Phenolic antioxidant [ppm] | AO-60 | 1,000 | 0 | 0 | 1,000 | 0 |
| Compounding agent [ppm] | | | 200 | 200 | 0 | 0 | 0 |
| YI | | | 0.57 | 0.43 | -0.14 | 1.22 | 2.07 |
| Haze | | | 0.20 | 0.15 | 0.17 | 0.26 | 0.26 |
| Total transmissivity [%] | | | 89.54 | 89.37 | 88.90 | 90.01 | 89.74 |
| Transmissivity [%] | 400 nm | | 88.30 | 88.28 | 86.71 | 88.28 | 86.44 |
| | 380 nm | | 86.83 | 86.93 | 82.61 | 87.31 | 84.45 |

Example 16

PCb: Trade name lupizeta T-3700 (viscosity-average molecular weight Mv: 17,500) manufactured by Mitsubishi Gas Chemical Company, Inc., which was a bisphenol A-type aromatic polycarbonate having a p-t-octylphenyl group as a terminal structure.

[Table 9]

| | | | Example 16-1 | Example 16-2 | Comparative example 13-1 | Comparative example 13-2 | Comparative example 13-3 |
|---|---|---|---|---|---|---|---|
| Resin | | | PCb | PCb | PCb | PCb | PCb |
| Mold release agent [ppm] | | S-100A | 1,000 | 1,000 | 1,000 | 1,000 | 1,000 |
| Antioxidant | Phosphite-based antioxidant [ppm] | PEP-36 | 500 | 500 | 500 | 500 | 0 |
| | Phenolic antioxidant [ppm] | AO-60 | 1,000 | 0 | 0 | 1,000 | 0 |
| Compounding agent [ppm] | | | 200 | 200 | 0 | 0 | 0 |
| YI | | | 0.66 | 0.51 | -0.05 | 1.32 | 2.07 |
| Haze | | | 0.26 | 0.19 | 0.23 | 0.20 | 0.18 |
| Total transmissivity [%] | | | 89.46 | 89.33 | 88.66 | 90.06 | 89.71 |
| Transmissivity [%] | | 400 nm | 87.82 | 87.87 | 85.35 | 88.05 | 86.19 |
| | | 380 nm | 85.97 | 85.99 | 79.44 | 86.76 | 83.78 |

Example 17

PCc: Trade name lupizeta T-1380 (viscosity-average molecular weight Mv: 25,500) manufactured by Mitsubishi Gas Chemical Company, Inc., which was a bisphenol A-type aromatic polycarbonate having a p-benzoic acid hexadecane group as a terminal structure.

[Table 10]

| | | | Example 17 | Comparative example 14-1 | Comparative example 14-2 | Comparative example 14-3 |
|---|---|---|---|---|---|---|
| Resin | | | PCc | PCc | PCc | PCc |
| Mold release agent [ppm] | | S-100A | 1,000 | 1,000 | 1,000 | 1,000 |
| Antioxidant | Phosphite-based antioxidant [ppm] | PEP-36 | 500 | 500 | 500 | 0 |
| | Phenolic antioxidant [ppm] | AO-60 | 0 | 0 | 1,000 | 0 |
| Compounding agent [ppm] | | | 200 | 0 | 0 | 0 |
| YI | | | 1.42 | 0.99 | 1.72 | 2.01 |
| Haze | | | 0.26 | 0.31 | 0.28 | 0.29 |
| Total transmissivity [%] | | | 89.45 | 89.00 | 89.69 | 89.51 |

(continued)

| | | Example 17 | Comparative example 14-1 | Comparative example 14-2 | Comparative example 14-3 |
|---|---|---|---|---|---|
| Transmissivity [%] | 400 nm | 85.99 | 84.63 | 87.14 | 86.50 |
| | 380 nm | 82.28 | 78.56 | 85.83 | 85.18 |

Example 18

PCd: FPC-0210 (viscosity-average molecular weight Mv: 11,500) manufactured by Mitsubishi Gas Chemical Company, Inc., which was an aromatic polycarbonate obtained by an interfacial polymerization method using bisphenol AP as the starting material and para-tert-butylphenol as the chain terminator.

[Table 11]

| | | | Example 18 | Comparative example 15-1 | Comparative example 15-2 | Comparative example 15-3 |
|---|---|---|---|---|---|---|
| Resin | | | PCd | PCd | PCd | PCd |
| Mold release agent [ppm] | | S-100A | 1,000 | 1,000 | 1,000 | 1,000 |
| Antioxidant | Phosphite-based antioxidant [ppm] | PEP-36 | 500 | 500 | 500 | 0 |
| | Phenolic antioxidant [ppm] | AO-60 | 0 | 0 | 1,000 | 0 |
| Compounding agent [ppm] | | | 200 | 0 | 0 | 0 |
| YI | | | 2.47 | 2.16 | 3.26 | 4.65 |
| Haze | | | 0.55 | 0.83 | 1.14 | 0.69 |
| Total transmissivity [%] | | | 87.67 | 86.33 | 86.83 | 86.89 |
| Transmissivity [%] | 400 nm | | 77.98 | 74.51 | 79.38 | 75.34 |
| | 380 nm | | 70.06 | 62.53 | 74.53 | 67.99 |

Although all of improvement of the transmissivity, lowering of the YI value, and lowering of the haze value were not always realized in the polycarbonate resin compositions of Examples 15-18 compared to the corresponding comparative examples, but generally better properties were observed.

## Claims

**1.** A thermoplastic resin composition comprising a compounding agent represented by General formula (1) below:

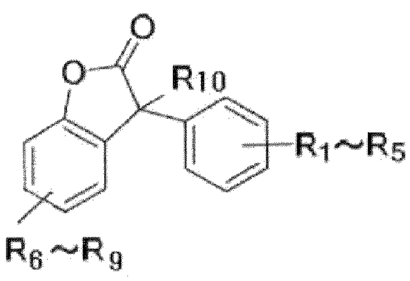

(1)

(in General formula (1),

$R_1$-$R_5$ each independently represent a hydrogen atom or an optionally substituted alkyl group with a total of 1-20 carbon atoms,
$R_6$-$R_9$ each independently represent a hydrogen atom or an optionally substituted alkyl group with a total of 1-20 carbon atoms, and
$R_{10}$ represents a hydrogen atom or an alkyl group with a total of 1-5 carbon atoms).

**2.** The thermoplastic resin composition according to Claim 1, further comprises a thermoplastic resin, and the thermoplastic resin comprises a spiroglycol-derived structural unit.

**3.** The thermoplastic resin composition according to Claim 2, wherein the weight-average molecular weight (Mw) of the thermoplastic resin in terms of polystyrene is 10,000-300,000.

**4.** The thermoplastic resin composition according to any one of Claims 1 to 3, further comprising an antioxidant,

wherein the antioxidant is optionally a phenolic antioxidant and/or a phosphite-based antioxidant, and/or
wherein the antioxidant is optionally contained in an amount of 1-10,000 ppm by weight based on the total weight of the resin composition.

**5.** The thermoplastic resin composition according to any one of Claims 1 to 4, wherein the compounding agent is contained in an amount of 1-10,000 ppm by weight based on the total weight of the resin composition.

**6.** The thermoplastic resin composition according to any one of Claims 1 to 5,

wherein the value of transmissivity (%) at a wavelength of 370-400 nm according to JIS K7105 is higher than that of a reference resin composition having the same composition but without the compounding agent, by 2.0 (%) or more, and/or
wherein the value of transmissivity (%) at a wavelength of 370-400 nm according to JIS K7105 is 1.1 times or more higher than that of a reference resin composition having the same composition but without the compounding agent.

**7.** The thermoplastic resin composition according to any one of Claims 1 to 6, wherein the amount of a volatile component generated upon heating at 250°C for 5 minutes is less than that of a reference resin composition having the same composition but without the compounding agent, and
the volatile component is any of formaldehyde, acetaldehyde, acetone, 2,3-butanedione, acetic acid, and formic acid; and/or
wherein the YI value according to JIS K7105 of the thermoplastic resin composition is lower than that of a reference resin composition having the same composition but without the compounding agent, by 0.20 or more.

**8.** The thermoplastic resin composition according to any one of Claims 1 to 7, wherein, in General formula (1) above,

three of $R_1$-$R_5$ are hydrogen atoms while two are alkyl groups,
two of $R_6$-$R_9$ are hydrogen atoms while two are alkyl groups, and
$R_{10}$ is a hydrogen atom; and/or

wherein, in General formula (1) above, the substituent is any of a halogen, a cyano group, an alkenyl group, an alkynyl group, and an alkoxy group.

**9.** The thermoplastic resin composition according to any one of Claims 1 to 8, further comprising a thermoplastic resin, wherein the thermoplastic resin is selected from the group consisting of a polycarbonate resin, a polyester resin, a polyester carbonate resin, a cycloolefin-based resin, and an acrylic resin, wherein the thermoplastic resin optionally is a polycarbonate resin, a polyester resin, or a polyester carbonate resin, which comprises a structural unit (B) derived from a monomer represented by General formula (2) below and/or a structural unit (C) derived from a monomer represented by General formula (3) below:

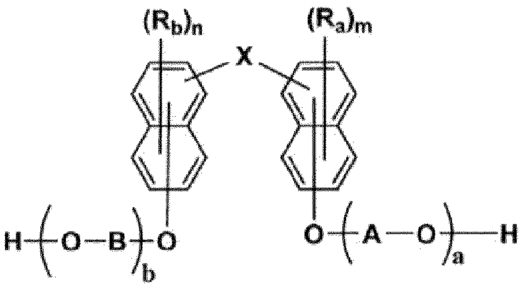
(2)

(in General formula (2),

$R_a$ and $R_b$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, an optionally substituted aryl group with 6-20 carbon atoms, an optionally substituted heteroaryl group with 6-20 carbon atoms containing one or more heteroatoms selected from O, N and S, an optionally substituted aryloxy group with 6-20 carbons, and $-C{\equiv}C-R_h$,
$R_h$ represents an optionally substituted aryl group with 6-20 carbon atoms or an optionally substituted heteroaryl group with 6-20 carbon atoms containing one or more heteroatoms selected from O, N and S,
X represents a single bond or an optionally substituted fluorene group,
A and B each independently represent an optionally substituted alkylene group with 1-5 carbon atoms,
m and n each independently represent an integer from 0 to 6, and
a and b each independently represent an integer from 0-10); and

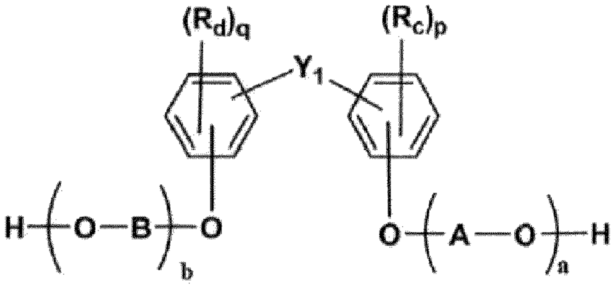
(3)

(in General formula (3),
$R_c$ and $R_d$ are each independently selected from the group consisting of a hydrogen atom, a halogen atom, an optionally substituted alkyl group with 1-20 carbon atoms, an optionally substituted alkoxyl group with 1-20 carbon atoms, an optionally substituted cycloalkyl group with 5-20 carbon atoms, an optionally substituted cycloalkoxyl group with 5-20 carbon atoms, and an optionally substituted aryl group with 6-20 carbon atoms,
A and B each independently represent an optionally substituted alkylene group with 1-5 carbon atoms,
p and q each independently represent an integer from 0 to 4,
a and b each independently represent an integer from 0-10,
Yi is a single bond, an optionally substituted fluorene group, or any one of the structural formulae represented by the General formulae (4) to (9) and (12) to (14) below:

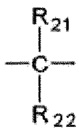
(4)

$-S-$
(5)

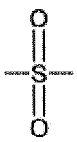
(6)

$-(CH_2)_r-$
(7)

$-O-$
(8)

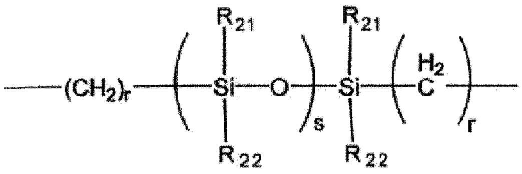

(9)

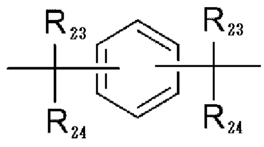

(1 2)

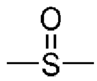

(1 3)

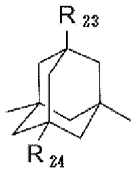

(1 4)

(in General formulae (4) to (9),

$R_{21}$ and $R_{22}$ each independently represent a hydrogen atom, a halogen atom, an optionally substituted alkyl group with 1-20 carbon atoms, or an optionally substituted aryl group with 6-30 carbon atoms, or an optionally substituted carbon or heterocyclic ring with 1-20 carbon atoms which is formed of $R_{21}$ and $R_{22}$ bonding to each other, and
r and s each independently represent an integer from 0 to 5,000; and
in General formulae (12) to (14),
$R_{23}$ and $R_{24}$ each independently represent a hydrogen atom, fluorine, chlorine, bromine, iodine, an optionally substituted alkyl group with 1-9 carbon atoms, an optionally substituted alkoxy group with 1-5 carbon atoms, an optionally substituted alkenyl group with 2-12 carbon atoms, or an optionally substituted aryl group with 6-12 carbon atoms.)

**10.** The thermoplastic resin composition according to Claim 9, wherein the weight-average molecular weight (Mw) of the thermoplastic resin in terms of polystyrene is 15,000-100,000.

**11.** The thermoplastic resin composition according to any one of Claims 1 to 10, further comprising a catalyst deactivator and/or a mold release agent.

**12.** The thermoplastic resin composition according to any one of Claims 1 to 11, comprising a compounding agent represented by General formula (1) above, which has peaks at diffraction angles $2\theta$ of 6.7 ± 0.2°, 10.4 ± 0.2°, 11.1 ± 0.2°, 12.7 ± 0.2°, 13.2 ± 0.2°, 15.2 ± 0.2°, 16.1 ± 0.2°, 17.3 ± 0.2°, 20.8 ± 0.2°, and 23.6 ± 0.2° in a powder X-ray diffraction pattern using Cu-Kα radiation, and/or
wherein the compounding agent of General formula (1) is represented by Formula (10) or (11) below

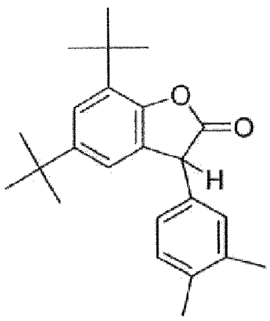

(1 0)

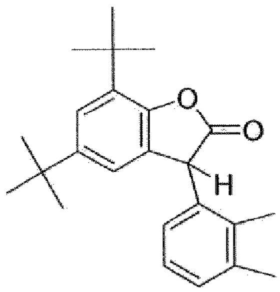

(1 1)

**13.** Use of the compounding agent represented by General formula (1) as defined in Claims 1 and 8 to improve the value of transmissivity (%) of a thermoplastic resin composition at a wavelength of 370 nm-400 nm,
wherein the compounding agent is preferably represented by Formula (10) or (11) below

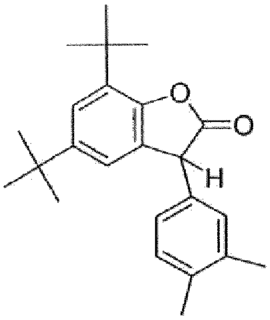

(10)

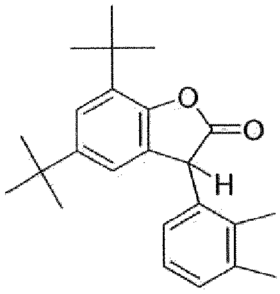

(11)

**14.** A method for producing a thermoplastic resin composition, the method comprising a step of adding a compounding agent represented by General formula (1) below to a thermoplastic resin:

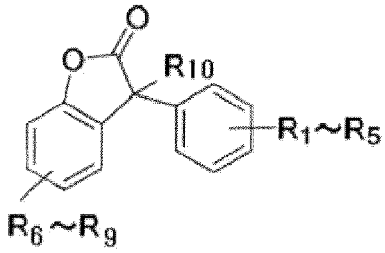

(1)

(in General formula (1),

$R_1$-$R_5$ each independently represent a hydrogen atom or an optionally substituted alkyl group with a total of 1-20 carbon atoms,
$R_6$-$R_9$ each independently represent a hydrogen atom or an optionally substituted alkyl group with a total of 1-20 carbon atoms, and
$R_{10}$ represents a hydrogen atom or an alkyl group with a total of 1-5 carbon atoms.

**15.** The method for producing a thermoplastic resin composition according to Claim 14, wherein the thermoplastic resin composition further comprises a thermoplastic resin, and the thermoplastic resin comprises a spiroglycol-derived structural unit.

**16.** The method for producing a thermoplastic resin composition according to Claim 15, wherein the weight-average molecular weight (Mw) of the thermoplastic resin in terms of polystyrene is 10,000-300,000.

[Figure 1]

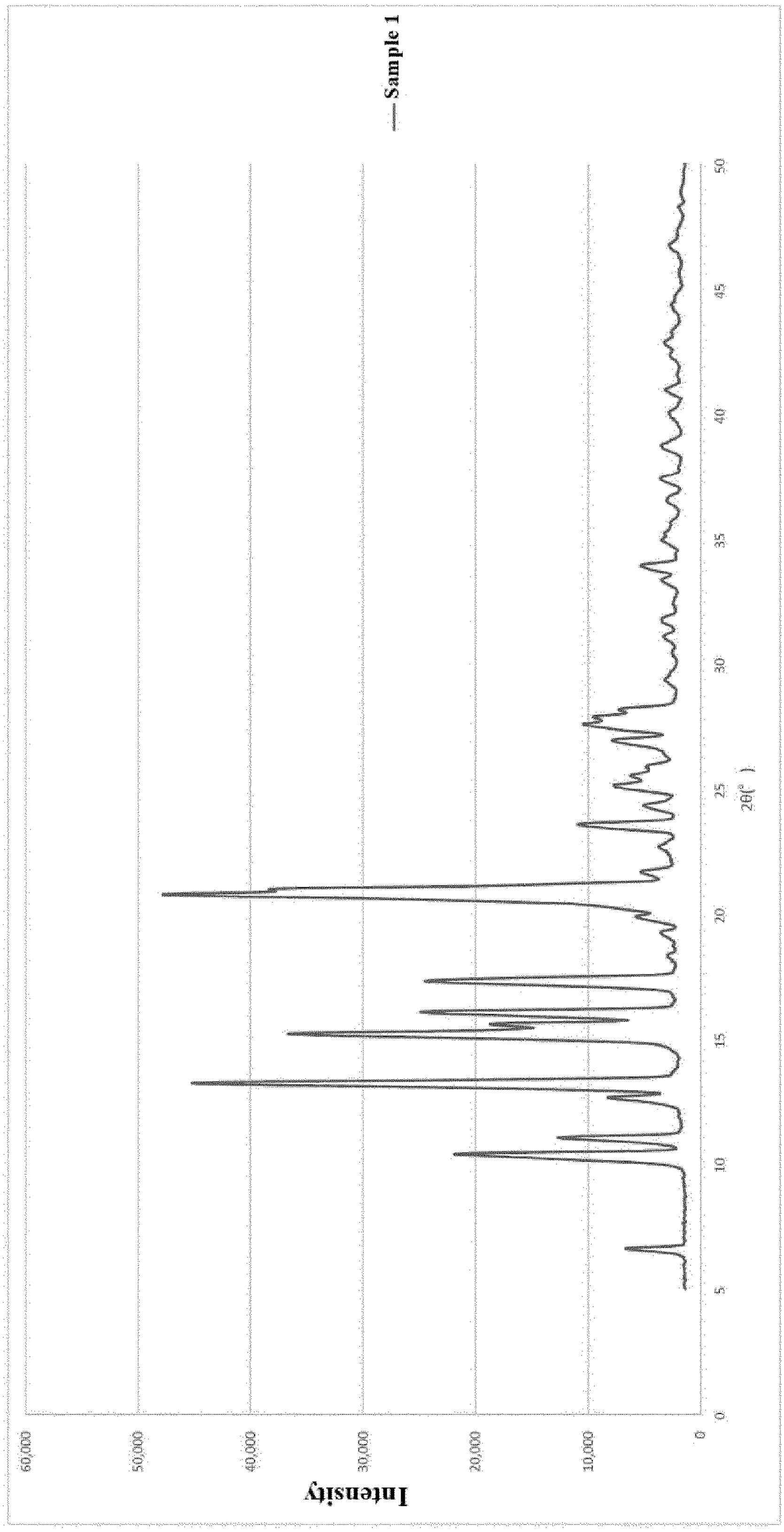
Sample 1
Intensity
60,000
50,000
40,000
30,000
20,000
10,000
0
0
5
10
15
20
25
30
35
40
45
50
2θ(° )

[Figure 2]

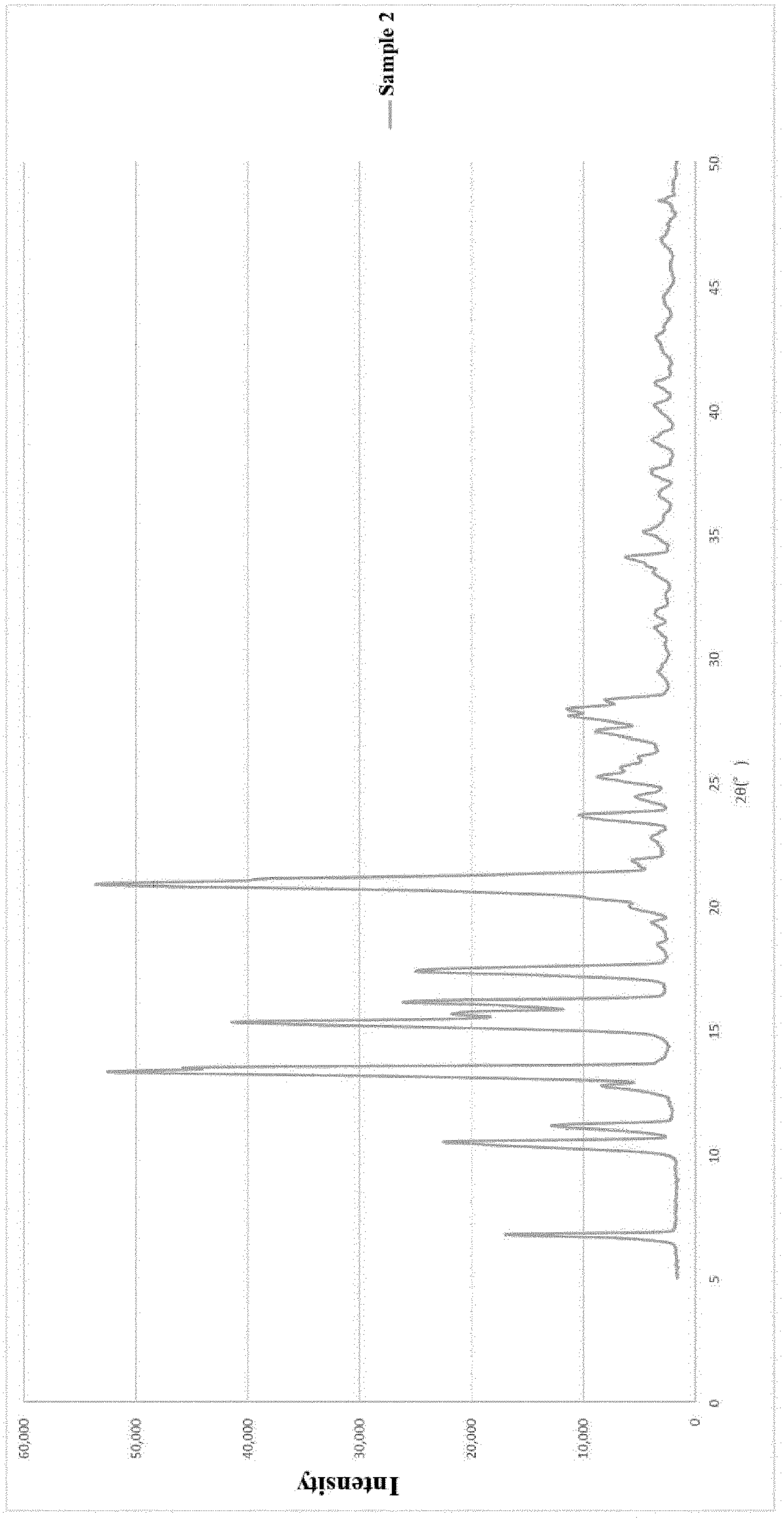
Sample 2
Intensity
60,000
50,000
40,000
30,000
20,000
10,000
0
0
5
10
15
20
25
30
35
40
45
50
2θ(°)

[Figure 3]

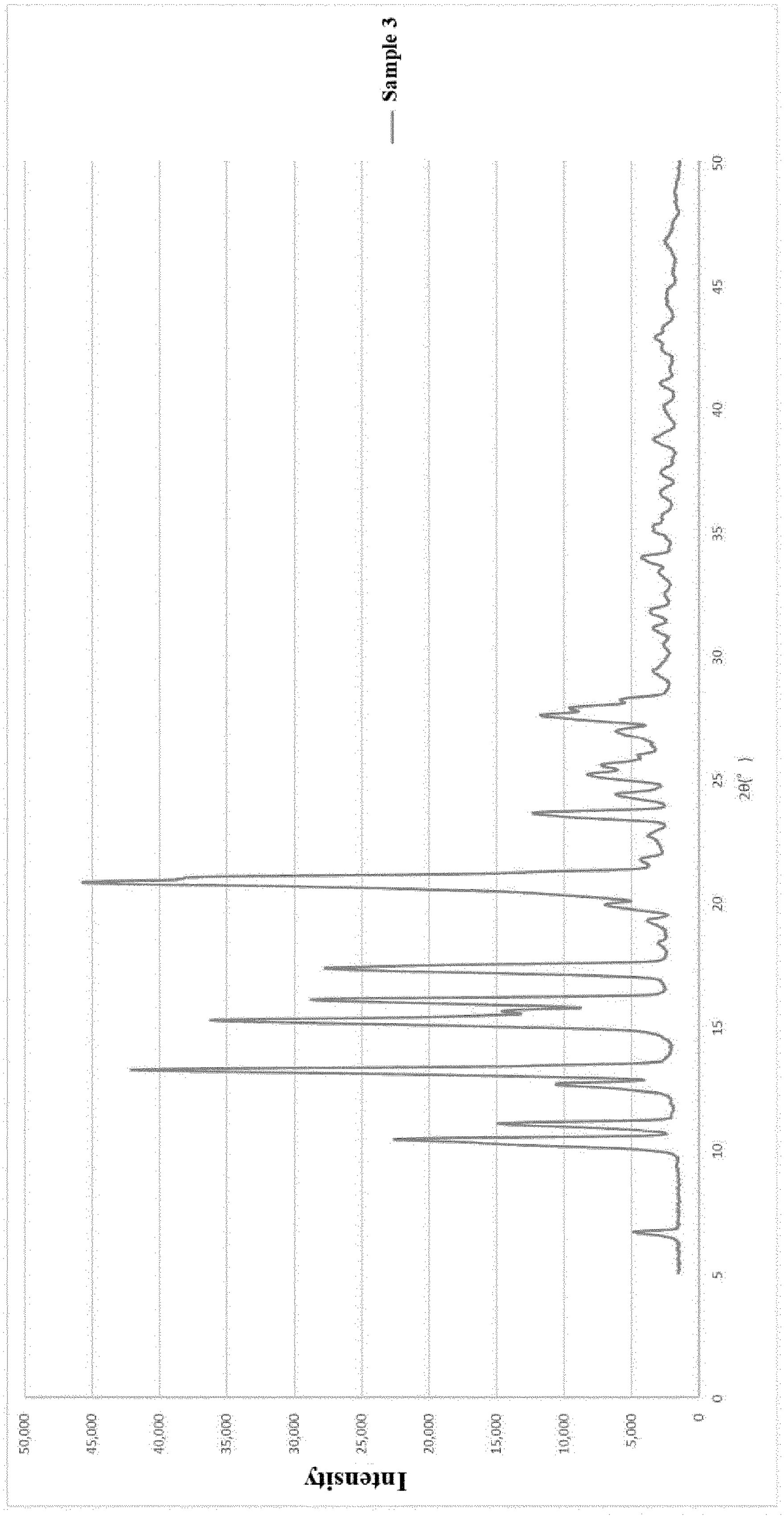
Sample 3
Intensity
2θ(°)
50,000
45,000
40,000
35,000
30,000
25,000
20,000
15,000
10,000
5,000
0
5
10
15
20
25
30
35
40
45
50

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9967232 A